(19) Europäisches Patentamt
European Patent Office
Office européen des brevets

(11) **EP 1 200 042 B1**

(12) **EUROPÄISCHE PATENTSCHRIFT**

(45) Veröffentlichungstag und Bekanntmachung des Hinweises auf die Patenterteilung:
**02.11.2005 Patentblatt 2005/44**

(21) Anmeldenummer: **00949370.1**

(22) Anmeldetag: **18.07.2000**

(51) Int Cl.[7]: **A61K 7/00**, A61K 7/48

(86) Internationale Anmeldenummer:
**PCT/EP2000/006847**

(87) Internationale Veröffentlichungsnummer:
**WO 2001/005361 (25.01.2001 Gazette 2001/04)**

(54) **EMULGATORFREIE FEINDISPERSE SYSTEME VOM TYP WASSER-IN-ÖL**

EMULSIFIER-FREE FINELY DISPERSED SYSTEMS OF THE OIL-IN-WATER TYPE

SYSTEMES A FINE DISPERSION DU TYPE EAU-DANS-HUILE EXEMPTS D'EMULSIFIANT

(84) Benannte Vertragsstaaten:
**AT BE CH CY DE DK ES FI FR GB GR IE IT LI LU MC NL PT SE**

(30) Priorität: **20.07.1999 DE 19934012**
**23.08.1999 DE 19939848**

(43) Veröffentlichungstag der Anmeldung:
**02.05.2002 Patentblatt 2002/18**

(73) Patentinhaber: **Beiersdorf AG**
**20253 Hamburg (DE)**

(72) Erfinder:
- **GERS-BARLAG, Heinrich**
  **D-25495 Kummerfeld (DE)**
- **MÜLLER, Anja**
  **D-23843 Rümpel (DE)**
- **STÄB, Franz**
  **D-21379 Echem (DE)**
- **SCHÖNROCK, Uwe**
  **D-23866 Nahe (DE)**

(56) Entgegenhaltungen:
**EP-A- 0 716 847**
**WO-A-96/28136**
**DE-A- 19 755 504**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Die vorliegende Erfindung betrifft emulgatorfreie feindisperse Systeme vom Typ Wasser-in-Öl, bevorzugt als kosmetische oder dermatologische Zubereitungen. Insbesondere betrifft die vorliegende Erfindung kosmetische und dermatologische Zubereitungen mit einem wirksamen. Schutz vor schädlichen Oxidationsprozessen in der Haut, aber auch zum Schutze kosmetischer Zubereitungen selbst bzw. zum Schutze der Bestandteile kosmetischer Zubereitungen vor schädlichen Oxidationsprozessen.

Darüber hinaus betrifft die Erfindung auch kosmetische und dermatologische Zubereitungen, welche Antioxidantien enthalten. In einer bevorzugten Ausführungsform betrifft die vorliegende Erfindung kosmetische und dermatologische Zubereitungen zur Prophylaxe und Behandlung kosmetischer oder dermatologischer Hautveränderungen wie z. B. der Hautalterung, insbesondere der durch oxidative Prozesse hervorgerufenen Hautalterung.

Die vorliegende Erfindung betrifft dementsprechend in bevorzugten Ausführungsformen kosmetische bzw. dermatologische Zubereitungen, enthaltend Wirkstoffe zur Pflege und zum Schutze der Haut, insbesondere der empfindlichen Haut wie auch ganz besonders im Vordergrunde stehend der durch intrinsische und/oder extrinsische Faktoren gealterten oder alternden Haut.

Die vorliegende Erfindung betrifft ferner kosmetische und dermatologische Lichtschutzformulierungen. Weiterhin betrifft die vorliegende Erfindung Zubereitungen zur kosmetischen und dermatologischen Behandlung oder Prophylaxe erythematöser, entzündlicher, allergischer oder autoimmunreaktiver Erscheinungen, insbesondere Dermatosen.

Die schädigende Wirkung des ultravioletten Teils der Sonnenstrahlung auf die Haut ist allgemein bekannt. In Abhängigkeit von ihrer jeweiligen Wellenlänge haben die Strahlen verschiedene Wirkungen auf das Organ Haut: Die sogenannte UV-C-Strahlung mit einer Wellenlänge, die kleiner als 290 nm ist, wird von der Ozonschicht in der Erdatmosphäre absorbiert und hat daher keine physiologische Bedeutung. Dagegen verursachen Strahlen im Bereich zwischen 290 nm und 320 nm, dem sogenannten UV-B-Bereich, ein Erythem einen einfachen Sonnenbrand oder sogar mehr oder weniger starke Verbrennungen. Als ein Maximum der Erythemwirksamkeit des Sonnenlichtes wird der engere Bereich um 308 nm angegeben.

Zum Schutz gegen UV-B-Strahlung sind zahlreiche Verbindungen bekannt, bei denen es sich zumeist um Derivate des 3-Benzylidencamphers, der 4-Aminobenzoesäure der Zimtsäure, der Salicylsäure, des Benzophenons sowie auch des 2-Phenylbenzimidazols handelt.

Man hat lange Zeit fälschlicherweise angenommen, daß die langwellige UV-A-Strahlung mit einer Wellenlänge zwischen 320 nm und 400 nm nur eine vemachlässigbare biologische Wirkung aufweist und daß dementsprechend die UV-B-Strahlen für die meisten Lichtschäden an der menschlichen Haut verantwortlich seien. Inzwischen ist allerdings durch zahlreiche Studien belegt daß UV-A-Strahlung im Hinblick auf die Auslösung photodynamischer, speziell phototoxischer Reaktionen und chronischer Veränderungen der Haut weitaus gefährlicher als UV-B-Strahlung ist. Auch kann der schädigende Einfluß der UV-B-Strahlung durch UV-A-Strahlung noch verstärkt werden.

So ist es u.a. erwiesen, daß selbst die UV-A-Strahlung unter ganz normalen Alltagsbedingungen ausreicht, um innerhalb kurzer Zeit die Collagen- und Elastinfasern zu schädigen, die für die Struktur und Festigkeit der Haut von wesentlicher Bedeutung sind. Hierdurch kommt es zu chronischen lichtbedingten Hautveränderungen - die Haut "altert" vorzeitig. Zum klinischen Erscheinungsbild der durch Licht gealterten Haut gehören beispielsweise Falten und Fältchen sowie ein unregelmäßiges, zerfurchtes Relief. Ferner können die von lichtbedingter Hautalterung betroffenen Partien eine unregelmäßige Pigmentierung aufweisen. Auch die Bildung von braunen Flecken, Keratosen und sogar Karzinomen bzw. malignen Melanomen ist möglich. Eine durch die alltägliche UV-Beiastung vorzeitig gealterte Haut zeichnet sich außerdem durch eine geringere Aktivität der Langerhanszellen und eine leichte chronische Entzündung aus.

Ferner können bereits sehr geringe Strahlendosen photochemische Reaktionen auslösen. Hierzu gehört insbesondere die Bildung freier Radikale, welche wiederum aufgrund ihrer hohen Reaktivität unkontrollierte Folgereaktionen auslösen können. Um solchen Reaktionen vorzubeugen, können den kosmetischen bzw. dermatologischen Formulierungen zusätzlich Antioxidantien und/oder Radikalfänger zugesetzt werden. So ist beispielsweise vorgeschlagen worden, Vitamin E, eine Substanz mit bekannter antioxidativer Wirkung in Lichtschutzformulierungen einzusetzen, allerdings bleibt hier die erzielte Wirkung weit hinter der erhofften zurück.

Etwa 90 % der auf die Erde gelangenden ultravioletten Strahlung besteht aus UV-A-Strahlen. Während die UV-B-Strahlung in Abhängigkeit von zahlreichen Faktoren stark variiert (z.B. Jahres- und Tageszeit oder Breitengrad), bleibt die UV-A-Strahlung unabhängig von jahres- und tageszeitlichen oder geographischen Faktoren Tag für Tag relativ konstant. Gleichzeitig dringt der überwiegende Teil der UV-A-Strahlung in die lebende Epidermis ein, während etwa 70 % der UV-B-Strahlen von der Hornschicht zurückgehalten werden.

Ein Sonnenbad wird von den meisten Menschen als angenehm empfunden, die nachteiligen Folgen zunächst nicht beachtet. Allerdings hat sich in den letzten Jahren durchaus ein Bewußtsein über die negativen Auswirkungen einer zu intensiven Sonnenbestrahlung herausgebildet, weshalb mehr und stärker schützende Sonnenschutzmittel

angewendet werden.

Da die Beiträge der verschiedenen Wellenlängebereiche des UV-Lichtes zu lichtbedingten Hautveränderungen nicht vollständig geklärt sind, geht man heute verstärkt davon aus, daß vorbeugender Schutz sowohl gegen UV-A- als auch gegen UV-B-Strahlen, beispielsweise durch Auftrag von Lichtschutzfiltersubstanzen in Form einer kosmetischen oder dermatologischen Formulierung auf die Haut, von grundsätzlicher Wichtigkeit ist. Kosmetische oder dermatologische Mittel sollen, in dünner Schicht auf die Haut aufgetragen, diese vor den negativen Auswirkungen der Sonnenstrahlung schützen.

Im allgemeinen ist das Lichtabsorptionsverhalten von Lichtschutzfiltersubstanzen sehr gut bekannt und dokumentiert, zumal in den meisten Industrieländern Positivlisten für den Einsatz solcher Substanzen existieren, welche recht strenge Maßstäbe an die Dokumentation anlegen. Je nachdem, in welchem Bereich des UV-Lichtes absorbiert wird, unterscheidet man UV-B-Filter, UV-A-Filter und Breitbandfilter (welche über den gesamten Bereich des UV-A und UV-B eine Filterwirkung zeigen). Durch entsprechende Auswahl des UV-Filters und seiner Konzentration im Sonnenschutzmittel hat man die Möglichkeit, den Grad der Abschirmung des UV-Lichtes zu beeinflussen. Für die Dosierung der Substanzen in den fertigen Formulierungen können die Extinktionswerte allerdings allenfalls eine Orientierungshilfe bieten, denn durch Wechselwirkungen mit Inhaltsstoffen der Formulierung oder der Haut selbst können Unwägbarkeiten auftreten. Ferner ist in der Regel schwierig vorab abzuschätzen, wie gleichmäßig und in welcher Schichtdicke die Filtersubstanz in und auf der Hornschicht der Haut verteilt ist.

Die Wirksamkeit von Sonnenschutzmittel bzw. der ihnen zugrunde liegenden UV-Filter wird in der Regel in biologischen Wirksamkeitsprüfungen unter standardisierten Bedingungen bestimmt.

Der Lichtschutzfaktor (LSF, oft auch SPF (sun protection factor) genannt) gibt die Verlängerung der Sonnenbestrahlung an, die durch Verwendung des Sonnenschutzmittels ermöglicht wird. Er ist der Quotient aus Erythemschwellenzeit mit Sonnenschutzmittel und Erythemschwellenzeit ohne Sonnenschutzmittel.

Zur Prüfung der UV-A-Schutzleistung wird üblicherweise die IPD-Methode verwendet (IPD ≡ immediate pigment darkening). Hierbei wird - ähnlich der Bestimmung des Lichtschutzfaktors - ein Wert ermittelt, der angibt, um wieviel länger die mit dem Lichtschutzmittel geschützte Haut mit UV-A-Strahlung bestrahlt werden kann, bis die gleiche Pigmentierung auftritt wie bei der ungeschützten Haut.

Eine andere, europaweit etablierte Prüfungsmethode ist der Australische Standard AS/NZS 2604:1997. Dabei wird die Absorption der Zubereitung im UV-A-Bereich gemessen. Um den Standard zu erfüllen, muß die Zubereitung mindestens 90 % der UV-A-Strahlung im Bereich 320 -360 nm absorbieren.

Die meisten Sonnenschutzmittel werden in Wassernähe oder bei sportlicher Betätigung (Schwitzen) angewendet, weshalb der Wasserfestigkeit solcher Formulierungen besondere Bedeutung beizumessen ist. Ein wasserfestes Sonnenschutzmittel schützt den Anwender nicht nur nach dem Baden, sondern bewahrt ihn auch während des Badens vor einem Sonnenbrand.

Um hohe Lichtschutzfaktoren bei gleichzeitiger sehr guter Wasserfestigkeit zu erreichen, sind W/O-Formulierungen von Vorteil. Allerdings weisen W/O-Emulsionen des Standes der Technik häufig unbefriedigende kosmetische Eigenschaften auf. Bei der Anwendung können entsprechende Zubereitungen auf der Haut einen fettigen, glänzenden und zum Teil klebrigen Eindruck hinterlassen und sich - insbesondere auf behaarter Haut - schwierig verteilen lassen. Im Einzelfall können daher derartige Zubereitungen sogar nicht vermarktungsfähig sein, da sie vom Verbraucher nicht akzeptiert bzw. negativ beurteilt werden.

Eine Aufgabe der vorliegenden Erfindung war daher, den Nachteilen des Standes der Technik Abhilfe zu schaffen und Zubereitungen vom Typ Wasser-in-Öl zu finden, welche beispielsweise die Formulierung hoher Lichtschutzfaktoren bei gleichzeitiger sehr guter Wasserfestigkeit erlauben, welche aber auf der Haut keinen schmierigen oder klebrigen Eindruck hinterlassen.

Lichtdermatosen werden in der Regel durch UV-A-Strahlung ausgelöst. Die häufigste aller Photodermatosen ist die polymorphe Lichtdermatose (PLD), welche u. a. auch als Sonnenallergie oder Mallorca-Akne bezeichnet wird und in der Literatur (z. B. *A. Voelckel et al, Zentralblatt Haut- und Geschlechtskrankheiten (1989), 156*, *S.2*) eine Vielzahl von weiteren Bezeichnungen trägt.

Über 20 % der weißen Bevölkerung in den Industriestaaten der nördlichen Hemisphäre sind von der PLD betroffen. Bei den Patienten kommt es im Bereich der sonnenexponierten Hautareale (Gesicht, Hals, Dekollete sowie Arme und Beine) in der Regel nach der ersten intensiveren Sonnenbestrahlung im Frühjahr zunächst zu Juckreiz und zur Ausbildung einer flächenhaften Hautrötung, im weiteren zeitlichen Verlauf können ferner kleine Knötchen, Plaques, Bläschen oder Papeln auftreten.

Erythematöse Hauterscheinungen treten auch als Begleiterscheinungen bei gewissen Hauterkrankungen oder -unregelmäßigkeiten auf. Beispielsweise ist der typische Hautausschlag beim Erscheinungsbild der Akne regelmäßig mehr oder weniger stark gerötet.

Aufgabe der Erfindung war es dementsprechend auch, kosmetische dermatologische und pharmazeutische Zubereitungen sowie Lichtschutzformulierungen zu schaffen, die zur Prophylaxe und Behandlung lichtempfindlicher Haut, insbesondere Photodermatosen, bevorzugt PLD dienen.

Antioxidantien werden hauptsächlich als Schutzsubstanzen gegen den Verderb der sie enthaltenden Zubereitungen verwendet. Dennoch ist bekannt, daß auch in der menschlichen und tierischen Haut unerwünschte Oxidationsprozesse auftreten können. Solche Prozesse spielen eine wesentliche Rolle bei der Hautalterung.

Die chronologische Hautalterung wird z. B. durch endogene, genetisch determinierte Faktoren verursacht. In Epidermis und Dermis kommt es alterungsbedingt z. B. zu folgenden Strukturschäden und Funktionsstörungen, die auch unter den Begriff "Senile Xerosis" fallen können:

a) Trockenheit, Rauhigkeit und Ausbildung von Trockenheitsfältchen,
b) Juckreiz und
c) verminderte Rückfettung durch Talgdrüsen (z. B. nach Waschen).

Exogene Faktoren, wie UV-Licht und chemische Noxen, können kumulativ wirksam sein und z. B. die endogenen Alterungsprozesse beschleunigen bzw. sie ergänzen. In Epidermis und Dermis kommt es insbesondere durch exogene Faktoren z. B. zu folgenden Strukturschäden- und Funktionsstörungen in der Haut, die über Maß und Qualität der Schäden bei chronologischer Alterung hinausgehen:

d) Sichtbare Gefäßerweiterungen (Teleangiektasien, Cuperosis);
e) Schlaffheit und Ausbildung von Falten;
f) lokale Hyper-, Hypo- und Fehlpigmentierungen (z. B. Altersflecken) und
g) vergrößerte Anfälligkeit gegenüber mechanischem Stress (z. B. Rissigkeit).

Die vorliegende Erfindung betrifft insbesondere Produkte zur Pflege der auf natürliche Weise gealterten Haut, sowie zur Behandlung der Folgeschäden der Lichtalterung, insbesondere der unter a) bis g) aufgeführten Phänomene.

Produkte zur Pflege gealterter Haut sind an sich bekannt. Sie enthalten z. B. Retinoide (Vitamin A-Säure und/ oder deren Derivate) bzw. Vitamin A und/oder dessen Derivate. Ihre Wirkung auf die Strukturschäden ist allerdings umfangsmäßig begrenzt. Daüber hinaus gibt es bei der Produktentwicklung erhebliche Schwierigkeiten die Wirkstoffe in ausreichendem Maße gegen oxidativen Zerfall zu stabilisieren. Die Verwendung Vitamin A-Säure-haltiger Produkte bedingt_ darüber hinaus oft starke erythematöse Hautreizungen. Retinoide sind daher nur in geringen Konzentrationen einsetzbar.

Die Verwendung von Flavonen bzw. Flavonoiden in der Kosmetik bzw. Dermatologie ist an sich bekannt. So beschreibt die DE-44 44 238-A1 Kombinationen von Zimtsäurederivaten und Flavonglycosiden, beispielsweise $\alpha$-Glycosylrutin als Antioxidantien und als Wirkstoffe gegen andere Indikationen. Ferner beschreibt die Schrift JP-OS Hei-04-363,395 ein Verfahren, um die Zersetzung von Parfumbestandteilen zu verhindern, welche sich unter anderem durch einen Zusatz an $\alpha$-Glucosylrutin zu den entsprechenden Zubereitungen auszeichnet. Ferner beschreiben die Schriften EP-OS 586 303 und EP-OS 595 694 die Verwendung von Flavonoiden als Antioxidantien bzw Lichtschutzsubstanzen in Kosmetika.

Ein erheblicher Nachteil von Flavonderivaten. Flavanonderivaten bzw. Flavonoiden ist allerdings, daß sie eine gewisse Instabilität aufweisen. Ein weiterer Nachteil dieser Substanzen liegt darin begründet, daß sie die Produkte, in welchen sie eingesetzt werden, in höheren Konzentrationen gelblich färben, insbesondere wenn sie in Formulierungen eingesetzt werden, welche Titandioxid enthalten. Da der Verbraucher eine Gelbfärbung als unschön empfindet, kann dies im Einzelfall dazu führen, daß derartige Zubereitungen nicht vermarktungsfähig sein können da sie vom Verbraucher nicht akzeptiert bzw. negativ beurteilt werden.

Eine weitere Aufgabe der vorliegenden Erfindung bestand also darin, den geschilderten Nachteilen des Standes der Technik abzuhelfen bzw. diese zumindest zu lindern.

Unter Emulsionen versteht man im allgemeinen heterogene Systeme, die aus zwei nicht oder nur begrenzt miteinander mischbaren Flüssigkeiten bestehen, die üblicherweise als Phasen bezeichnet werden. In einer Emulsion ist eine der beiden Flüssigkeiten in Form feinster Tröpfchen in der anderen Flüssigkeit dispergiert.

Um die dauerhafte Dispergierung einer Flüssigkeit in einer anderen zu erreichen, ist bei Emulsionen im herkömmlichen Sinn der Zusatz einer grenzflächenaktiven Substanz (Emulgator) notwendig. Emulgatoren weisen einen amphiphilen Molekülaufbau auf, bestehend aus einem polaren (hydrophilen) und einem unpolaren (lipophilen) Molekülteil, die räumlich voneinander getrennt sind. Emulgatoren setzen die Grenzflächenspannung zwischen den Phasen herab, indem sie an der Grenzfläche zwischen beiden Flüssigkeiten angeordnet sind. Sie bilden an der Phasengrenze Öl/Wasser Grenzflächenfilme aus, wodurch dem irreversiblen Zusammenfließen der Tröpfchen entgegengewirkt wird. Zur Stabilisierung von Emulsionen werden häufig Emulgatorgemische verwendet.

Herkömmliche Emulgatoren können entsprechend ihrem hydrophilen Molekülteil in ionische (anionische, kationische und amphotere) und nichtionische untergliedert werden:

- Das wohl bekannteste Beispiel eines anionischen Emulgators ist die Seife, als die man gewöhnlich die wasserlöslichen Natrium- oder Kaliumsalze der gesättigten und ungesättigten höheren Fettsäuren bezeichnet.
- Wichtige Vertreter der kationischen Emulgatoren sind die quartären Ammonium-Verbindungen.

- Der hydrophile Molekülteil nichtionischer Emulgatoren besteht häufig aus Glycerin, Polyglycerin, Sorbitanen, Kohlenhydraten bzw. Polyoxyethylenglykolen und ist meistens über Ester- und Etherbindungen mit dem lipophilen Molekülteil verknüpft. Dieser besteht üblicherweise aus Fettalkoholen, Fettsäuren oder Isofettsäuren.

Durch Variation der Struktur und der Größe des polaren und des unpolaren Molekülteils lassen sich Lipophilie und Hydrophilie von Emulgatoren in weiten Grenzen verändern.

Entscheidend für die Stabilität einer Emulsion ist die richtige Auswahl der Emulgatoren. Dabei sind die Charakteristiken aller im System enthaltenen Stoffe zu berücksichtigen. Betrachtet man z. B. Hautpflegeemulsionen, so führen polare Ölkomponenten und beispielsweise UV-Filter zu Instabilitäten. Neben den Emulgatoren werden daher noch andere Stabilisatoren verwendet, die beispielsweise die Viskosität der Emulsion erhöhen und/oder als Schutzkolloid wirken.

Emulsionen stellen den wichtigsten Produkttyp im Bereich kosmetischer und/oder dermatologischer Zubereitungen dar.

An sich ist die Verwendung der üblichen Emulgatoren in kosmetischen oder dermatologischen Zubereitungen unbedenklich. Dennoch können Emulgatoren, wie letztlich jede chemische Substanz, im Einzelfalle allergische oder auf Überempfindlichkeit des Anwenders beruhende Reaktionen hervorrufen. Es hat daher nicht an Versuchen gefehlt, die Menge an üblichen Emulgatoren auf ein Minimum, im Idealfall sogar vollständig zu reduzieren.

Eine Reduktion der benötigten Emulgatormenge kann z. B. erreicht werden, wenn ausgenutzt wird, daß feinstverteilte Feststoffteilchen eine zusätzlich stabilisierende Wirkung haben. Dabei kommt es zu einer Anreicherung des festen Stoffes an der Phasengrenze Öl/Wasser in Form einer Schicht, wodurch Zusammenfließen der dispersen Phasen verhindert wird. Von wesentlicher Bedeutung sind dabei nicht die chemischen, sondern die Oberflächeneigenschaften der Feststoffpartikel.

Eine relativ neue technische Entwicklung ist es, kosmetische oder dermatologische Zubereitungen nur durch feinstverteilte Feststoffteilchen zu stabilisieren.. Solche "emulgatorfreien" Emulsionen werden nach ihrem Erfinder auch als Pickering-Emulsionen bezeichnet.

Eine Möglichkeit, eine Feststoffstabilisierung in einer kosmetischen oder dermatologischen Zubereitung vorzunehmen, ist nach May-Alert (*Pharmazie in unserer Zeit, 15. Jahrg. 1986, Nr. 1, 1-7*) beispielsweise, Emulgatorgemische zu verwenden, die sowohl anionische als auch kationische Tenside enthalten. Da beim Zusammengeben von Anion- und Kationtensiden immer unlösliche, elektroneutrale Verbindungen ausfallen, läßt sich durch gezieltes Ausfällen dieser neutralen Tenside in der Grenzfläche Öl/Wasser eine zusätzliche Feststoffstabilisierung im Sinne einer Pickering-Emulsion erreichen.

Ferner beschreibt die WO-Schrift WO-98/42301 emulgatorfreie feindisperse Systeme vom Typ Wasser-in-Öl, welche durch den Zusatz mikronisierter, anorganischer Pigmente stabilisiert werden, die aus der Gruppe der Metalloxide, insbesondere Titandioxid gewählt werden.

Allerdings konnte der Stand der Technik nicht den Weg zur vorliegenden Erfindung weisen.

Eine weitere Aufgabe der vorliegenden Erfindung war, den Stand der Technik um kosmetische oder dermatologische Zubereitungen zu bereichern, in welchen auf jeglichen Einsatz von Emulgatoren herkömmlicher Art verzichtet werden kann.

Erstaunlicherweise werden alle diese Aufgabe gelöst durch

kosmetische oder dermatologische Zubereitungen, die feindisperse Systeme vom Typ Wasser-in-Öl darstellen, enthaltend

1. eine Ölphase,
2. eine Wasserphase,
3. mindestens ein modifiziertes Schichtsilikat, welches sowohl hydrophile als auch lipophile Eigenschaften zeigt, welches also amphiphilen Charakter besitzt und sich an der Grenzfläche Wasser/Öl anordnet,
4. mindestens ein Flavon, Flavanon und/oder Flavonoid und
5. höchstens 0,5 Gew.-% eines oder mehrerer Emulgatoren

sowie
gegebenenfalls weitere kosmetische oder pharmazeutische Hilfs-, Zusatz- und/oder Wirkstoffe enthaltend.

Es ist erfindungsgemäß besonders vorteilhaft, wenn die Zubereitungen deutlich weniger als 0,5 Gew.-% eines oder mehrerer Emulgatoren enthalten. Ganz besonders bevorzugt sind erfindungsgemäße Zubereitungen, welche gänzlich frei von Emulgatoren im herkömmlichen Sinn sind.

Die erfindungsgemäßen Zubereitungen stellen Abmischungen aus Ölen bzw. öllöslichen Substanzen und Wasser bzw. wasserlöslichen Komponenten dar, die durch den Zusatz der modifizierten Schichtsilikatpartikel stabilisiert werden und keinen Emulgator im herkömmlichen Sinn enthalten müssen.

Die erfindungsgemäßen Zubereitungen sind in jeglicher Hinsicht überaus befriedigende Präparate, die erstaunlicherweise hervorragende kosmetische Eigenschaften zeigen und sich durch eine ausgezeichnete Hautverträglichkeit auszeichnen. Es war für den Fachmann nicht vorauszusehen gewesen, daß die erfindungsgemäßen Zubereitungen besser als Radikalfänger wirken, besser die Bindung von schädlichen Photoprodukten an Lipide, DNS und Proteine verhindern, besser gegen die Hautalterung wirken, besser die Haut gegen Photoreaktionen schützen und besser entzündlichen Reaktionen vorbeugen sowie weniger Gelbfärbung zeigen würden als Zubereitungen des Standes der Technik.

Es war darüber hinaus überraschend, daß in den erfindungsgemäßen Zubereitungen auf jeglichen Zusatz von Titandioxid und Zinkoxid verzichtet werden kann und daß in Lichtschutzzubereitungen gemäß der vorliegenden Erfindung ein hoher Lichtschutzfaktor und/oder eine hohe UV-A-Schutzleistung bei gleichzeitiger sehr guter Wasserfestigkeit erreicht werden kann.

**Flavone, Flavanone und Flavonoide**

Flavon und seine Derivate (oft auch kollektiv "Flavone" genannt) sind durch folgende Grundstruktur gekennzeichnet (Substitutionspostitionen angegeben):

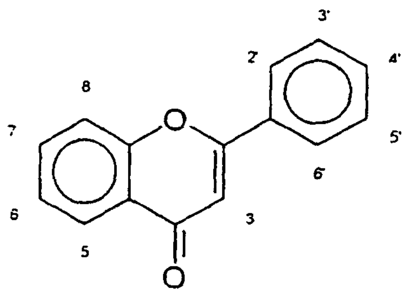

Einige der wichtigeren Flavone, welche auch in der belebten Natur aufzufinden sind, sind in der nachstehenden Tabelle aufgeführt:

| | OH-Substitutionspositionen | | | | | | | |
|---|---|---|---|---|---|---|---|---|
| | 3 | 5 | 7 | 8 | 2' | 3' | 4' | 5' |
| Flavon | - | - | - | - | - | - | - | - |
| Flavonol | + | - | - | - | - | - | - | - |
| Chrysin | - | + | + | - | - | - | - | - |
| Galangin | + | + | + | - | - | - | - | - |
| Apigenin | - | + | + | - | - | - | + | - |
| Fisetin | + | - | + | - | - | + | + | - |
| Luteolin | - | + | + | - | - | + | + | - |
| Kämpferol | + | + | + | - | - | - | + | - |
| Quercetin | + | + | + | - | - | + | + | - |
| Morin | + | + | + | - | + | - | + | - |
| Robinetin | + | - | + | - | - | + | + | + |
| Gossypetin | + | + | + | + | - | + | + | - |
| Myricetin | + | + | + | - | - | + | + | + |

In der Natur kommen Flavone in der Regel in glycosidierter Form vor.

Flavonoide sind Glycoside der Flavone, der Flavanone, deren Grundgerüst durch die folgende Struktur gekennzeichnet ist:

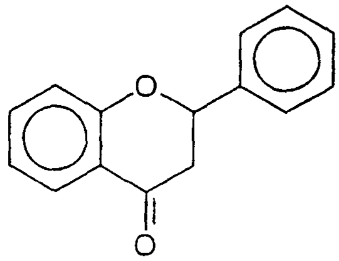

,

der 3-Hydroxyflavone (Flavonole), deren Grundgerüst durch die folgende Struktur gekennzeichnet ist:

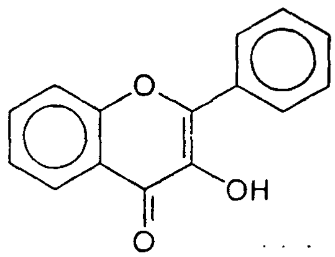

,

der Aurone, deren Grundgerüst durch die folgende Struktur gekennzeichnet ist:

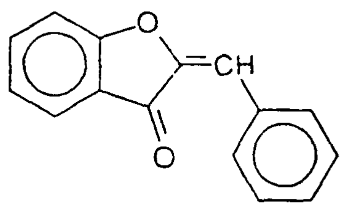

,

sowie der Isoflavone, deren Grundgerüst durch die folgende Struktur gekennzeichnet ist:

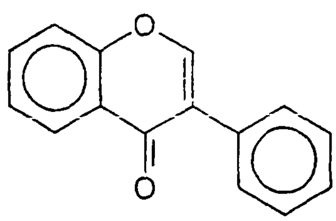

Erfindungsgemäß werden die Flavonoide bevorzugt gewählt gewählt aus der Gruppe der Substanzen der generischen Strukturformel

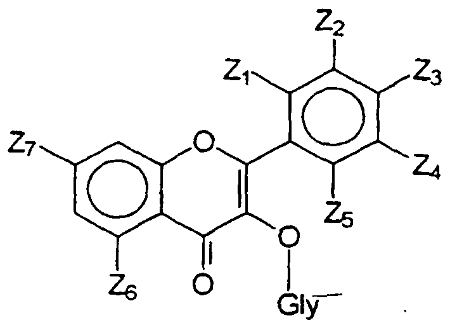

wobei $Z_1$ - $Z_7$ unabhängig voneinander gewählt werden aus der Gruppe H, OH, Alkoxysowie Hydroxyalkoxy-, wobei die Alkoxy- bzw. Hydroxyalkoxygruppen verzweigt und unverzweigt sein und 1 - 18 C-Atome aufweisen können, und wobei Gly gewählt wird aus der Gruppe der Mono- und Oligoglycosidreste.

Erfindungsgemäß können die Flavonoide aber auch vorteilhaft gewählt werden aus der Gruppe der Substanzen der generischen Strukturformel

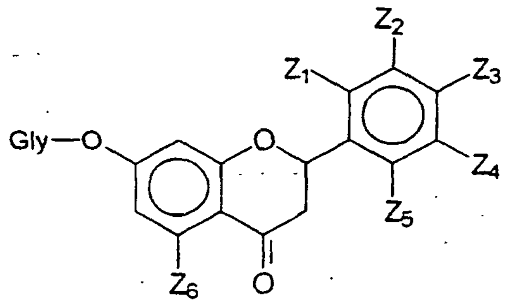

wobei $Z_1$ - $Z_6$ unabhängig voneinander gewählt werden aus der Gruppe H, OH, Alkoxysowie Hydroxyalkoxy-, wobei die Alkoxy- bzw. Hydroxyalkoxygruppen verzweigt und unverzweigt sein und 1 - 18 C-Atome aufweisen können, und wobei Gly gewählt wird aus der Gruppe der Mono- und Oligoglycosidreste.

Bevorzugt können solche Strukturen gewählt werden aus der Gruppe der Substanzen der generischen Strukturformel

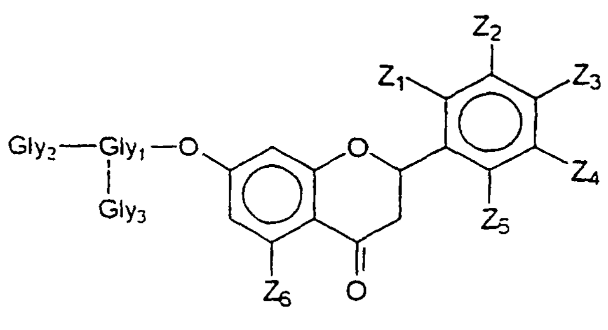

wobei $Gly_1$, $Gly_2$ und $Gly_3$ unabhängig voneinander Monoglycosidreste oder darstellen. $Gly_2$ bzw. $Gly_3$ können auch einzeln oder gemeinsam Absättigungen durch Wasserstoffatome darstellen.

Bevorzugt werden $Gly_1$, $Gly_2$ und $Gly_3$ unabhängig voneinander gewählt aus der Gruppe der Hexosylreste, insbesondere der Rhamnosylreste und Glucosylreste. Aber auch andere Hexosylreste, beispielsweise Allosyl, Altrosyl. Galactosyl, Gulosyl, Idosyl, Mannosyl und Talosyl sind gegebenenfalls vorteilhaft zu verwenden. Es kann auch erfindungsgemäß vorteilhaft sein, Pentosylreste zu verwenden.

Vorteilhaft werden $Z_1$ - $Z_5$ unabhangig voneinander gewählt aus der Gruppe H, OH, Methoxy-, Ethoxy- sowie 2-Hydroxyethoxy-, und die Flavonglycoside haben die Struktur

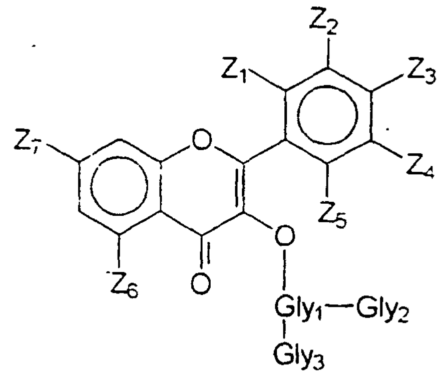

Besonders vorteilhaft werden die erfindungsgemäßen Flavonglycoside aus der Gruppe, welche durch die folgende Struktur wiedergegeben werden:

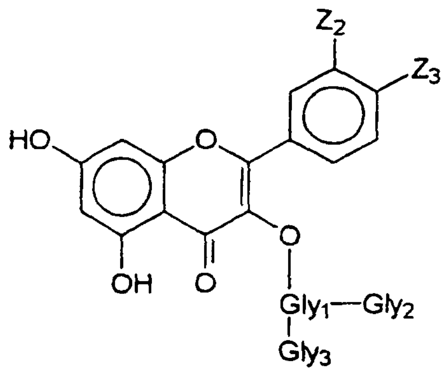

wobei $Gly_1$, $Gly_2$ und $Gly_3$ unabhängig voneinander Monoglycosidreste oder darstellen. $Gly_2$ bzw. $Gly_3$ können auch einzeln oder gemeinsam Absättigungen durch Wasserstoffatome darstellen.

Bevorzugt werden $Gly_1$, $Gly_2$ und $Gly_3$ unabhängig voneinander gewählt aus der Gruppe der Hexosylreste, insbesondere der Rhamnosylreste und Glucosylreste. Aber auch andere Hexosylreste, beispielsweise Allosyl, Altrosyl, Apiosyl, Biosidyl, Galactosyl, Gulosyl, Idosyl, Mannosyl, Talosyl, Ascorbinyl, Arabinsoyl, Dulcityl, Fructosyl, Mannityl, Rhamnosyl, Ribosyl, Sorbityl, Xylosyl, sind gegebenenfalls vorteilhaft zu verwenden. Es kann auch erfindungsgemäß vorteilhaft sein, Pentosylreste zu verwenden.

Besonders vorteilhaft im Sinne der vorliegenden Erfindung ist, das oder die Flavonglycoside zu wählen aus der Gruppe α-Glucosylrutin, α-Glucosylmyrictrin, α-Glucosylisoquercitriin und α-Glucosylquercitrin.

Ein erfindungsgemäß besonders vorteilhaftes Flavonoid ist α-Glucosylrutin. Es zeichnet sich durch folgende Struktur aus:

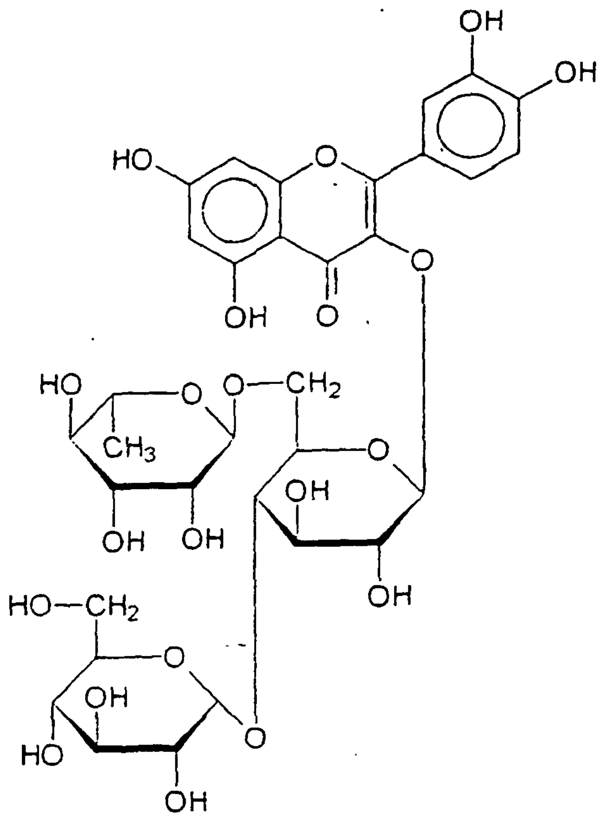

Ein weiteres erfindungsgemäß besonders vorteilhaftes Flavonoid ist Naringin (Aurantiin, Naringenin-7-rhamnoglucosid). Es zeichnet sich durch folgende Struktur aus:

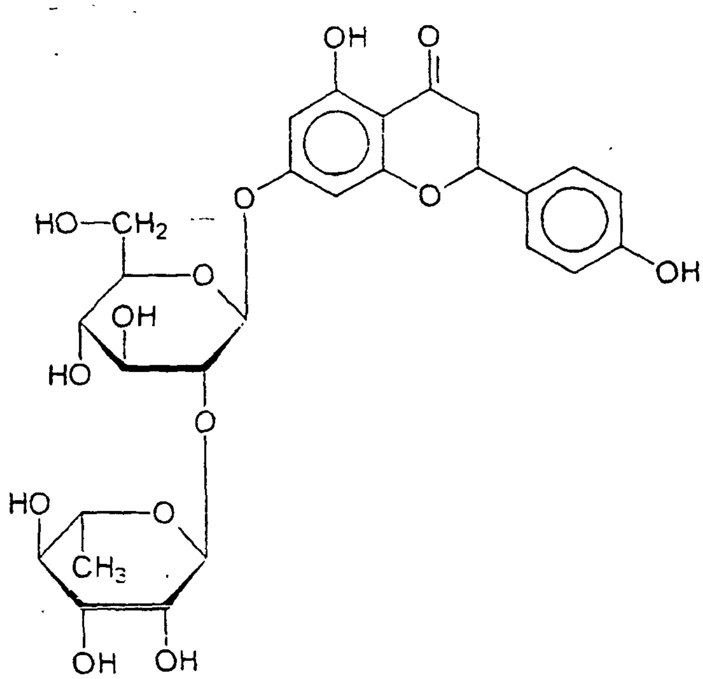

Ein weiteres erfindungsgemäß besonders vorteilhaftes Flavonoid ist Hesperidin (3',5,7-Tri-hydroxy-4'-methoxyflavanon-7-rutinosid, Hesperidosid, Hesperetin-7-O-rutinosid). Es zeichnet sich durch folgende Struktur aus:

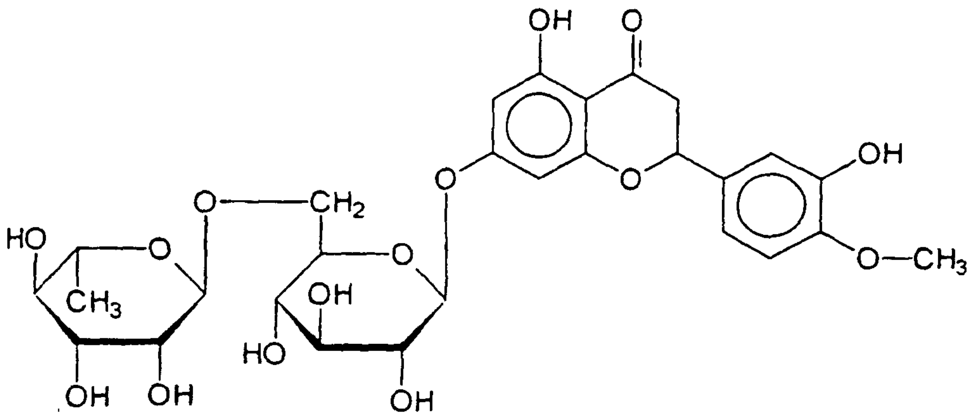

Ein weiteres erfindungsgemäß besonders vorteilhaftes Flavonoid ist Rutin (3,3',4',5,7-Pentahydroxyflavon-3-rutinosid, Quercetin-3-rutinosid, Sophorin, Birutan, Rutabion, Taurutin, Phytomelin, Melin). Es zeichnet sich durch folgende Struktur aus:

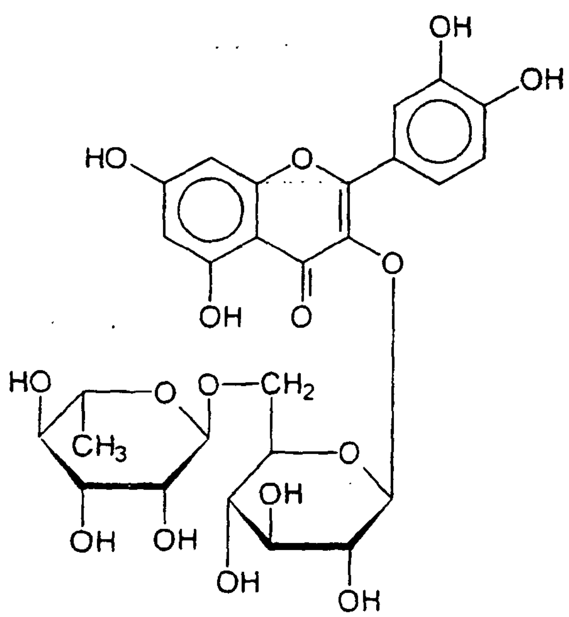

Ein weiteres erfindungsgemäß besonders vorteilhaftes Flavonoid ist Troxerutin (3,5-Dihydroxy-3',4',7-tris (2-hydroxyethoxy)-flavon-3-(6-O-(6-deoxy-α-L-mannopyranosyl)-β-D-glucopyranosid)). Es zeichnet sich durch folgende Struktur aus:

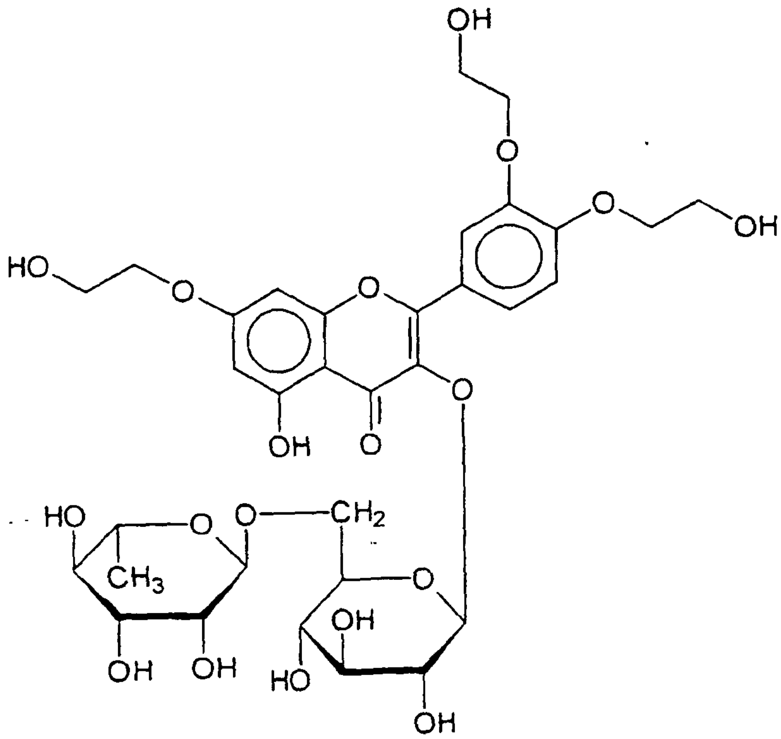

Ein weiteres erfindungsgemäß besonders vorteilhaftes Flavonoid ist Monoxerutin (3,3',4',5-Tetrahydroxy-7-(2-hydroxyethoxy)-flavon-3-(6-O-(6-deoxy-α-L-mannopyranosyl)-β-D-glucopyranosid)). Es zeichnet sich durch folgende Struktur aus:

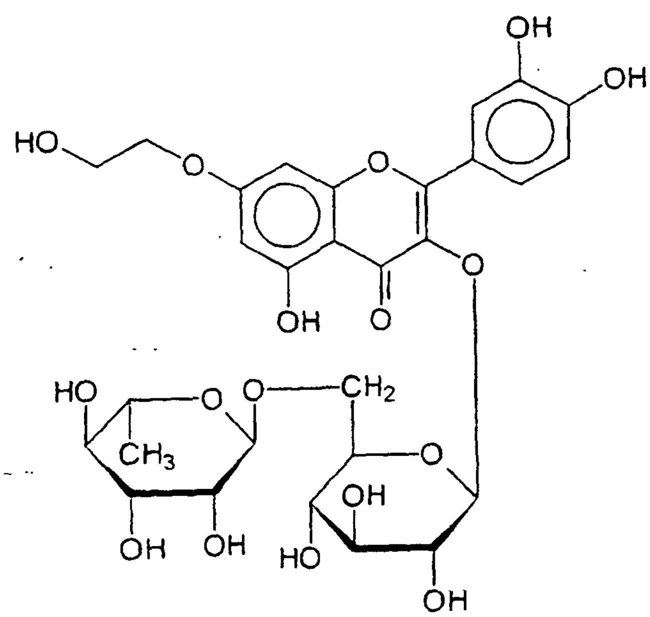

Ein weiteres erfindungsgemäß besonders vorteilhaftes Flavonoid ist Diosmin (3',5,7-Trihydroxy-4'methoxyflavon-7-rhamnoglucosid). Es zeichnet sich durch folgende Struktur aus:

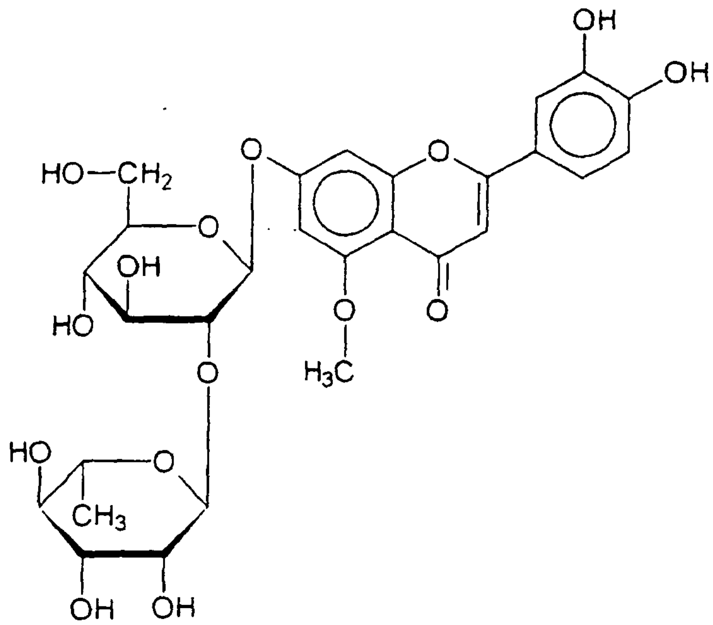

Es kann ebenso von Vorteil sein, Flavonoide zu verwenden, deren Glucosidreste über phenolische Hydroxyfunktionen des Flavons in den Positionen 3', 4' und/oder 5' gebunden ist oder sind.

Ferner kann es vorteilhaft sein, Flavonoidderivate zu verwenden, deren phenolische Gruppe in der Position 9 frei vorliegt. In diesen Fällen handelt es sich um sogenannte Chalkone. Die Struktur des namengebenden Vertreters dieser Substanzklasse ist wie folgt gekennzeichnet:

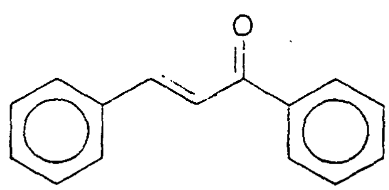

Ein vorteilhafter Vertreter dieser Substanzklasse ist Phlorizin (1-(2-(β-D-Glucopyranosyloxy)-4,6-dihydroxyphenyl)-3-(4-hydroxyphenyl)-1-propanon), welches durch folgende Struktur gekennzeichnet ist:

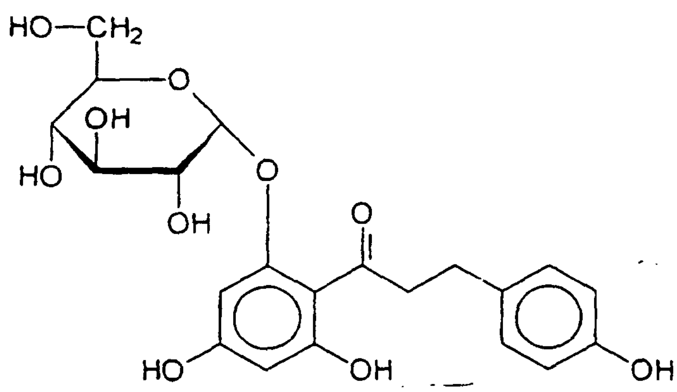

Ein weiterer vorteilhafter Vertreter dieser Substanzklasse ist Neohesperidindihydrochalkon (1-(4-((2-O-(6-deoxy-α-L-mannopyranosyl)-β-D-glucopyranosyl)oxy)-2,6-dihydroxyphenyl)-3-(3-hydroxy-4-methoxyphenyl)-1-propanon), welches durch folgende Struktur gekennzeichnet ist:

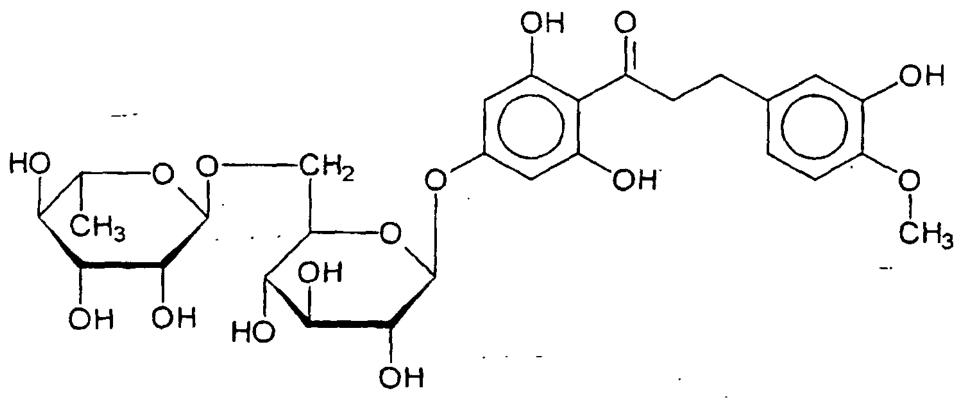

Die erfindungsgemäß verwendeten, kosmetisch oder pharmazeutisch unbedenklichen Flavonderivate und Flavanonderivate, insbesondere Flavonoide im eigentlichen Sinne, werden nachfolgend - ungeachtet, ob eine Einzelsubstanz oder ein Isomerengemisch oder ein Gemisch verschiedener Einzelsubstanzen vorliegt - kollektiv als "Flavonoide" bezeichnet.

Es kann auch vorteilhaft im Sinne der vorliegenden Erfindung sein, natürliche, das sind insbesondere pflanzliche Produkte, z. B. Pflanzenextrakte in die erfindungsgemäßen Zubereitungen einzuarbeiten, welche sich durch einen Gehalt an kosmetisch oder pharmazeutisch unbedenklichen Flavonoiden auszeichnen, beispielsweise nach üblichen Verfahren erhaltene wäßrige, wäßrig-alkoholische oder wäßrig-glycolische Extrakte wie auch trockene Extrakte.

Als insbesondere vorteilhaft haben sich erwiesen: Citrusfruchtschalenextrakt, Citrusfruchtkemextrakt, Sojaextrakt (z.B. das Handelsprodukt Phytodermin der Gesellschaft Chem.Laboratorium Dr.Kurt Richter GmbH), Sophora Japonica-Extrakt (z.B. das Handelsprodukt Sophorine der Gesellschaft Solabia), Frauendistelextrakt ((z.B. das Handelsprodukt Psoralen Silymarin der Gesellschaft Mani GmbH Chemische Produkte), Katzenpfötchenblütenextrakt. Spinatextrakt und ein gemischter Pflanzenextrakt aus Passionsblumen, schwarzen Johannisbeeren und Weinblättern (z. B. das Handelsprodukt AE Complex der Gesellschaft Solabia), Calendulaextrakt (z.B. das Handelsprodukt Pot Marigold AMI watersoluble der Gesellschaft Alban Muller).

Die Gesamtmenge an einem oder mehreren Flavonen. Flavanonen und/oder Flavonoiden in den fertigen kosmetischen oder dermatologischen Zubereitungen wird vorteilhaft aus dem Bereich von 0,01 bis 5.0 Gew -%. bevorzugt 0.1 bis 2.0 Gew.-% gewählt, bezogen auf das Gesamtgewicht der Zubereitungen.

**Modifizierte Schichtsilikate**

Silikate sind Salze und Ester (Kieselsäureester) der Orthokieselsäure $[Si(OH)_4]$ und deren Kondensationsprodukte. Die Silikate sind nicht nur die artenreichste Klasse der Mineralien, sondern auch geologisch und technisch außerordentlich wichtig. Über 80 % der Erdkruste bestehen aus Silikaten. Schichtsilikate (Phyllosilikate, Blattsilikate) sind (idealerweise) Silikat-Strukturen mit zweidimensional unendlichen Schichten aus $[SiO_4]^{4-}$-Tetraedern, wobei jedes Tetraeder über 3 Brucken-Sauerstoffe mit Nachbar-Tetraedern verbunden ist.

Chemische Formeln lassen sich für Schichtsilikate nur angenähert aufstellen, da sie ein großes Ionenaustausch-Vermögen besitzen und Silizium gegen Aluminium und dieses wiederum gegen Magnesium, $Fe^{2-}$, $Fe^{3-}$, Zn und dergleichen ausgetauscht werden kann. Die daraus möglicherweise resultierende negative Ladung der Schichten wird in der Regel durch Kationen, insbesondere durch $Na^-$ und $Ca^{2+}$ in Zwischenschicht-Positionen ausgeglichen.

Schichtsilikate können durch reversible Einlagerung von Wasser (in der 2- bis 7-fachen Menge) und anderen Substanzen wie z. B. Alkoholen, Glykolen und dergleichen mehr aufquellen: Ihre Verwendung als Verdickungsmittel in kosmetischen Mitteln ist dementsprechend an sich bekannt. Allerdings konnte der Stand der Technik nicht den Weg zur vorliegenden Erfindung weisen.

Vorteilhafte Schichtsilikate im Sinne der vorliegenden Erfindung sind beispielsweise solche, deren größte Ausdehnungsrichtung im unmodifizierten und ungequollenen Zustand im Mittel eine Länge von weniger als 10 µm hat. Beispielsweise können die mittleren Ausdehnungen der verwendeten modifizierten Schichtsilikatpartikel bei 1000 nm x 100 nm x 1 nm und darunter liegen. Die effektive Größe der modifizierten Schichtsilikatpartikel in einer kosmetischen oder dermatologischen Formulierung hängt selbstverständlich von der Menge an eingelagerten Substanzen ab.

Vorteilhafte modifizierte Schichtsilikate im Sinne der vorliegenden Erfindung sind beispielsweise modifizierte Smektite (Smectite).

Smektite sind stets sehr feinkörnige (meist < 2 mm), überwiegend als lamellenförmige, moosartige oder kugelförmige Aggregate vorkommende Dreischicht-Tonminerale (2:1-Schichtsilikate), in denen eine zentrale Schicht aus oktaedrisch koordinierten Kationen sandwichartig von 2 Schichten aus $[(Si,Al)O_4]$-Tetraedern umgeben ist. Smektite werden idealisiert durch die folgende Strukturformel beschrieben, worin weiß ausgefüllte Kreise Silizium- und/oder Aluminiumatome, hellgrau ausgefüllte Kreise Sauerstoffatome, dunkelgrau ausgefüllte Kreise Wasserstoffatome und schwarz ausgefüllte Kreise Aluminium-, Magnesium-, Eisenatome und/oder weitere Austauschkationen darstellen:

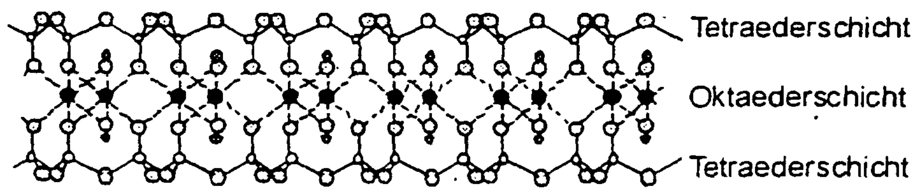


Vorteilhafte modifizierte Smektite sind z. B. modifizierte Montmorillonite. Montmorillonite werden durch die angenäherte chemische Formel $Al_2[(OH)_2/Si_4O_{10}] \cdot n\ H_2O$ bzw. $Al_2O_3 \cdot 4\ SiO_2 \cdot H_2O \cdot n\ H_2O$ beschrieben und stellen zu den dioktaedrischen Smektiten gehörende Tonmineralien dar.

Besonders vorteilhaft im Sinne der vorliegenden Erfindung sind ferner beispielsweise modifizierte Hektorite. Hektorite gehören zu den Smektiten und haben die angenäherte chemische Formel $M^+_{0.3}(Mg_{2.7}Li_{0.3})[Si_4O_{10}(OH)_2]$, worin $M^+$ meist $Na^+$ darstellt.

Vorteilhaft im Sinne der vorliegenden Erfindung sind ferner modifizierte Bentonite. Bentonite sind Tone und Gesteine, die Smektite, vor allem Montmorillonit, als Hauptminerale enthalten. Die "Roh"-Bentonite sind entweder Calcium-Bentonite (in Großbritannien als Fuller-Erden bezeichnet) oder Natrium-Bentonite (auch: Wyoming-Bentonite).

Modifizierte Schichtsilikate im Sinne der vorliegenden Erfindung sind Schichtsilikate, insbesondere die bereits genannten Schichtsilikattypen, deren Organophilie (auch: Lipophilie) - beispielsweise durch Umsetzung mit quarternären Ammonium-Verbindungen - erhöht wurde. Solche Schichtsilikate werden auch als organophile Schichtsilikate bezeichnet.

Besonders vorteilhaft im Sinne der vorliegenden Erfindung sind sogenannte Bentone, d. h. organische Derivate von Montmorilloniten (bzw. Bentoniten) und/oder Hectoriten, die durch lonenaustausch-Reaktionen mit Alkylammonium-Basen hergestellt werden.

Vorteilhafte modifizierte Schichtsilikate im Sinne der vorliegenden Erfindung sind beispielsweise durch Umsetzung von Schichtsilikaten mit Quatemium-18 erhältlich. Quatemium-18 ist eine Mischung von quaternären Ammoniumchloridsalzen, welche durch die folgende Strukturformel beschrieben werden:

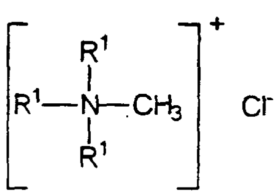

worin
die Reste $R^1$ unabhängig voneinander gewählt werden aus der Gruppe Methyl und hydrierte Talgreste mit einer Kettenlänge von 12 bis 20 Kohlenstoffatomen.

Erfindungsgemäß besonders bevorzugt sind Stearalkoniumhektorit, ein Reaktionsprodukt aus Hektorit und Stearalkoniumchlorid (Benzyldimethylstearylammoniumchlorid), und Quatemium-18 Hektorit, ein Reaktionsprodukt aus Hektorit und Quaternium-18, welche z. B. unter den Handelsbezeichnungen Bentone 27 und Bentone 38 bei Nordmann & Rassmann erhältlich sind.

Die Gesamtmenge an einem oder mehreren modifizierten Schichtsilikaten in den fertigen kosmetischen oder dermatologischen Zubereitungen wird vorteilhaft aus dem Bereich von 0.05 bis 20.0 Gew.-%, bevorzugt 0.1 bis 5.0 Gew.-% gewählt, bezogen auf das Gesamtgewicht der Zubereitungen.

Obwohl es besonders bevorzugt ist, die erfindungsgemäßen Zubereitungen nur durch den Zusatz von einem oder mehreren modifizierten Schichtsilikaten zu stabilisieren, kann es ferner vorteilhaft sein, die modifizierten Schichtsilikatpartikel mit weiteren amphiphilen Pigmenten zu kombinieren, welche gegebenenfalls auch zur Stabilisierung der Pickering-Emulsionen beitragen können.

Solche Pigmente sind beispielsweise mikronisierte, anorganische Pigmente, die gewählt werden aus der Gruppe der amphiphilen Metalloxide, insbesondere aus der Gruppe Titandioxid, Zinkoxid, Siliciumdioxid bzw. Silicate

(z. B. Talkum), wobei die Metalloxide sowohl einzeln als auch im Gemisch vorliegen können. Dabei ist es im wesentlichen unerheblich, in welcher der gegebenenfalls natürlich vorkommenden Modifikationen die verwendeten amphiphilen Metalloxide vorliegen.

Vorteilhaft ist es, den mittleren Partikeldurchmesser der zur Kombination mit modifizierten Schichtsilikaten verwendeten Pigmente zwischen 1 nm und 200 nm, besonders vorteilhaft zwischen 5 nm und 100 nm zu wählen.

Vorteilhaft im Sinne der vorliegenden Erfindung ist es, die erfindungsgemäßen modifizierten Schichtsilikate mit unbehandelten, nahezu reinen Pigmentpartikeln zu kombinieren, insbesondere mit solchen, welche auch als Farbstoff in der Lebensmittelindustrie und/oder als Absorber von UV-Strahlung in Sonnenschutzmitteln verwendet werden können. Vorteilhaft sind beispielsweise die bei der Firma Merck erhältlichen Zinkoxid-Pigmente sowie solche, die unter den Handelsbezeichnungen Zinkoxid neutral bei Haarmann & Reimer oder NanoX von der Harcros Chemical Group erhältlich sind.

Erfindungsgemäß vorteilhaft ist ferner die Kombination von modifizierten Schichtsilikaten mit anorganischen Pigmenten, die oberflächlich wasserabweisend behandelt ("gecoatet") sind, wobei gleichzeitig ein amphiphiler Charakter dieser Pigmente gebildet werden bzw. erhalten bleiben soll. Diese Oberflächenbehandlung kann darin bestehen, daß die Pigmente nach an sich bekannten Verfahren mit einer dünnen hydrophoben Schicht versehen werden.

Ein solches Verfahren, das im folgenden am Beispiel von Titandioxid beschrieben wird, besteht beispielsweise darin, daß die hydrophobe Oberflächenschicht nach einer Reaktion gemäß

$$n\ TiO_2 + m\ (RO)_3\ Si\text{-}R' \rightarrow n\ TiO_2\ (\text{oberfl.})$$

erzeugt wird n und m sind dabei nach Belieben einzusetzende stöchiometrische Parameter, R und R' die gewünschten organischen Reste. Besonders vorteilhafte Kombinationspartner sind $TiO_2$-Pigmente, beispielsweise die mit Aluminiumstearat beschichteten, unter der Handelsbezeichnung MT 100 T bei der Firma TAYCA erhältlichen.

Eine weitere vorteilhafte Beschichtung der Kombinationspartner besteht aus Dimethylpolysiloxan (auch: Dimethicone), einem Gemisch vollmethylierter, linearer Siloxanpolymere, die endständig mit Trimethylsiloxy-Einheiten blockiert sind. Besonders vorteilhaft im Sinne der vorliegenden Erfindung ist die Kombination von modifizierten Schichtsilikaten mit Zinkoxid-Pigmenten, die auf diese Weise beschichtet werden.

Vorteilhaft ist ferner, wenn die neben modifizierten Schichtsilikaten verwendeten anorganischen Pigmente mit einem Gemisch aus Dimethylpolysiloxan, insbesondere Dimethylpolysiloxan mit einer durchschnittlichen Kettenlänge von 200 bis 350 Dimethylsiloxan-Einheiten, und Silicagel beschichtet sind, welches auch als Simethicone bezeichnet wird. Es ist insbesondere von Vorteil, wenn die anorganischen Pigmente zusätzlich mit Aluminiumhydroxid bzw. Aluminiumoxidhydrat (auch: Alumina, CAS-Nr.: 1333-84-2) beschichtet sind. Besonders vorteilhafte Kombinationspartner sind Titandioxide, die mit Simethicone und Alumina beschichtet sind, wobei die Beschichtung auch Wasser enthalten kann. Ein Beispiel hierfür ist das unter dem Handelsnamen Eusolex T2000 bei der Firma Merck erhältliche Titandioxid.

Vorteilhaft im Sinne der vorliegenden Erfindung ist ferner die Kombination von modifizierten Schichtsilikaten mit einer Mischung verschiedener anorganischer, amphiphiler Pigmenttypen sowohl innerhalb eines Kristalls, beispielsweise als Eisenmischoxid oder Talkum (Magnesiumsilicat), als auch durch Mischung mehrerer Metalloxidtypen innerhalb einer Zubereitung. Besonders vorteilhafte Kombinationspartner sind Magnesiumsilicate, beispielsweise die unter der Handelsbezeichnung Talkum Micron bei der Firma Grolmann erhältlichen.

Die erfindungsgemäßen modifizierten Schichtsilikate können ferner vorteilhaft mit weiteren Pigmenten kombiniert werden, beispielsweise mit Titandioxidpigmenten, die mit Octylsilanol beschichtet sind, und/oder mit Siliciumdioxidpartikeln, die oberflächlich wasserabweisend behandelt sind. Zur Kombination geeignete Siliciumdioxidpartikel sind beispielsweise sphärische Polyalkylsilsesquioxan-Partikel wie sie in der Europäischen Offenlegungsschrift 0 686 391 erwähnt werden. Solche Polyalkylsilsesquioxan-Partikel sind beispielsweise unter den Handelsbezeichnungen Aerosil R972 und Aerosil 200V bei der Firma Degussa erhältlich.

Weiter vorteilhaft werden die modifizierten Schichtsilikate mit mikrofeinen Polymerpartikeln kombiniert, welche in der Zubereitung in Form von Feststoffen vorliegen. Günstige Kombinationspartner im Sinne der vorliegenden Erfindung sind beispielsweise Polycarbonate, Polyether, Polyethylen, Polypropylen, Polyvinylchlorid, Polystyrol, Polyamide, Polyacrylate und dergleichen mehr.

Erfindungsgemäß geeignete Kombinationspartner sind beispielsweise mikrofeine Polyamid-Partikel, insbesondere die unter der Handelsbezeichnung SP-500 bei der Firma TORAY erhältlichen. Ferner vorteilhaft sind Polyamid 6- (auch: Nylon 6) bzw. Polyamid 12- (auch: Nylon 12) Partikel. Polyamid 6 ist das aus ε-Aminocapronsäure (6-Aminohexansäure) oder ε-Caprolactam aufgebaute Polyamid [Poly(ε-caprolactam)], und Polyamid 12 ist ein Poly(ε-laurinlactam) aus ε-Laurinlactam. Vorteilhaft im Sinne der vorliegenden Erfindung sind beispielsweise Orgasol® 1002 (Polyamid 6) und Orgasol® 2002 (Polyamid 12) von der Firma ELF ATOCHEM.

Weitere vorteilhafte mikrofeine Polymerpartikel welche sich zur Kombination mit den erfindungsgemäßen

modifizierten Schichtsilikaten eignen, sind mikrofeine Polymethacrylate. Solche Partikel sind beispielsweise unter der Handelsbezeichnung POLYTRAP® bei der Firma DOW CHEMICAL erhältlich.

Es ist insbesondere vorteilhaft, wenngleich nicht zwingend, wenn die als Kombinationspartner verwendeten mikrofeinen Polymerpartikel oberflächlich beschichtet sind. Diese Oberflächenbehandlung kann darin bestehen, daß die Polymerpartikel nach an sich bekannten Verfahren mit einer dünnen hydrophilen Schicht versehen werden. Vorteilhafte Beschichtungen bestehen beispielsweise aus Titandioxid ($TiO_2$), Zirkoniumdioxid ($ZrO_2$) oder auch weiteren Polymeren, wie beispielsweise Polymethylmethacrylat. Besonders vorteilhafte mikrofeine Polymerpartikel im Sinne der vorliegenden Erfindung sind beispielsweise die nach dem in der US-Patentschrift 4,898,913 beschriebenen Verfahren zur hydrophilen Beschichtung hydrophober Polymerpartikel erhältlichen.

Der mittlere Partikeldurchmesser der als Kombinationspartner verwendeten mikrofeinen Polymerpartikel wird vorzugsweise kleiner als 100 µm, besonders vorteilhaft kleiner als 50 µm gewählt. Dabei ist es im wesentlichen unerheblich, in welcher Form (Plättchen, Stäbchen, Kügelchen etc.) die verwendeten Polymerpartikel vorliegen.

Die erfindungsgemäßen modifizierten Schichtsilikate werden ferner vorzugsweise mit amphiphilen modifizierten Polysacchariden kombiniert, welche keine verdickenden Eigenschaften zeigen.

Solche amphiphilen Polysaccharide sind beispielsweise durch Umsetzung von Stärke mit mono-, bi- oder polyfunktionellen Reagenzien bzw. Oxidations-Mitteln in weitgehend polymeranalog verlaufenden Reaktionen erhältlich.

Diese Reaktionen basieren im wesentlichen auf Umwandlungen der Hydroxy-Gruppen der Polyglucane durch Veretherung, Veresterung oder selektive Oxidation. Dabei entstehen z. B. sogenannte Stärkeether und Stärkeester der allgemeinen Strukturformel

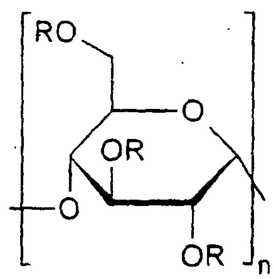

**Strukturformel (I)**

worin R beispielsweise ein Wasserstoff und/oder einen Alkyl- und/oder Aralkylrest (im Fall der Stärkeether) oder ein Wasserstoff und/oder einen organischen und/oder anorganischen Säure-Rest (im Fall der Stärkeester) darstellen kann. Stärkeether und Stärkeester sind vorteilhafte Kombinationspartner im Sinne der vorliegenden Erfindung

Besonders vorteilhaft ist es, die erfindungsgemäßen modifizierten Schichtsilikate mit Stärkeethern zu kombinieren, z. B. mit solchen, die durch Veretherung von Stärke mit Tetramethylolacetylendiharnstoff erhältlich sind und welche als Amylum non mucilaginosum (nicht quellende Stärke) bezeichnet werden.

Insbesondere vorteilhaft ist ferner die Kombination von erfindungsgemäßen modifizierten Schichtsilikate mit Stärkeestem und/oder deren Salzen, beispielsweise mit Natrium- und/oder Aluminiumsalzen niedrigsubstituierter Halbester der Stärke, insbesondere mit Natrium Stärke n-Octenylsuccinat der Strukturformel (I), worin R sich durch die folgende Struktur auszeichnet

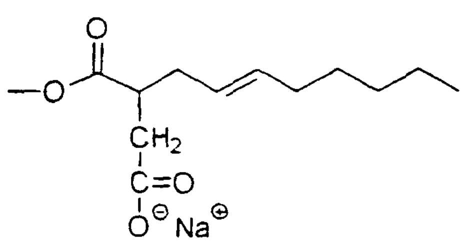

und welches z. B. unter der Handelsbezeichnung Amiogum® 23 bei der Firma CERESTAR erhältlich ist sowie mit Aluminium Stärke Octenylsuccinat, insbesondere mit den unter den Handelsbezeichnungen Dry Flo® Elite LL und Dry Flo® PC bei der Firma CERESTAR erhältlichen.

Vorteilhaft ist es, den mittleren Partikeldurchmesser der zur Kombination mit den erfindungsgemäßen modifizierten Schichtsilikaten verwendeten, modifizierten Polysaccharide kleiner als 20 μm, besonders vorteilhaft kleiner als 15 μm zu wählen.

Die Liste der genannten modifizierten Polysaccharide, die mit den modifizierten Schichtsilikaten kombiniert werden können, soll selbstverständlich nicht limitierend sein. Modifizierte Polysaccharide, die im Sinne der vorliegenden Erfindung vorteilhafte Kombinationspartner darstellen, sind auf zahlreichen, an sich bekannten Wegen, sowohl chemischer als auch physikalischer Natur erhältlich. Zur Herstellung solcher Polysaccharide sind auch neue Wege prinzipiell denkbar. Wesentlich dabei ist, daß die modifizierten Polysaccharide amphiphile Eigenschaften zeigen und daß sie nicht verdickend wirken.

Die erfindungsgemäßen modifizierten Schichtsilikate werden ferner vorzugsweise mit Bomitrid kombiniert.

Vorteilhafte Kombinationspartner im Sinne der vorliegenden Erfindung sind beispielsweise die im folgenden aufgelisteten Bornitride:

| Handelsname | erhältlich bei: |
|---|---|
| Boron Nitride Powder | Advanced Ceramics |
| Boron Nitride Powder | Sintec Keramik |
| Ceram Blanche | Kawasaki |
| HCST Boron Nitride | Stark |
| Très BN® | Carborundum |
| Wacker-Bornitrid BNP | Wacker-Chemie |

Vorteilhaft ist es, den mittleren Partikeldurchmesser der verwendeten Bornitridpartikel kleiner als 20 μm, besonders vorteilhaft kleiner als 15 μm zu wählen.

Ebenfalls vorteilhafte Kombinationspartner sind Bornitridpartikel, die oberflächlich wasserabweisend behandelt ("gecoatet") sind, wobei gleichzeitig der amphiphile Charakter gebildet werden bzw. erhalten bleiben soll.

Eine vorteilhafte Beschichtung der Bomitridpartikel besteht aus Dimethylpolysiloxan (auch: Dimethicone), einem Gemisch vollmethyliener, linearer Siloxanpolymere, die endständig mit Trimethylsiloxy-Einheiten blockiert sind. Vorteilhaft sind beispielsweise die bei der Firma Carborundum unter der Handelsbezeichnung Très BN® UHP 1106 erhältlichen, mit Dimethicone behandelten Bornitridpartikel.

Vorteilhaft ist ferner eine Beschichtung der Bornitridpartikel mit Polymethylhydrogensiloxan, einem linearen Polysiloxan, welches auch als Methicone bezeichnet wird. Vorteilhafte, mit Methicone behandelte Bomitridpartikel sind beispielsweise die bei der Firma Carborundum unter der Handelsbezeichnung Très BN® UHP 1107 erhältlichen.

Vorteilhaft in allen vorgenannten Fällen ist es, die Gesamtkonzentration aller Pigmente größer als 0,05 Gew.-%, besonders vorteilhaft zwischen 0,05 Gew.-% und 30 Gew.-% bezogen auf das Gesamtgewicht der Zubereitungen zu wählen, wobei die Konzentration an modifizierten Schichtsilikaten - ebenfalls bezogen auf das Gesamtgewicht der Zubereitungen - im Sinne der vorliegenden Erfindung vorzugsweise aus dem Bereich 0,05 Gew.-% bis 20 Gew.-%, vorteilhaft 0,1 Gew.-% bis 5 Gew.-% zu wählen ist.

Die erfindungsgemäßen Pickering-Emulsionen können als Grundlage für kosmetische oder dermatologische Formulierungen dienen. Diese können wie üblich zusammengesetzt sein und beispielsweise zur Behandlung und zur Pflege der Haut, als Lippenpflegeprodukt, als Deoprodukt und als Schmink- bzw. Abschminkprodukt in der dekorativen Kosmetik oder als Lichtschutzpräparat dienen. Zur Anwendung werden die erfindungsgemäßen kosmetischen und dermatologischen Zubereitungen in der für Kosmetika üblichen Weise auf die Haut in ausreichender Menge aufgebracht.

Entsprechend können kosmetische oder topische dermatologische Zusammensetzungen im Sinne der vorliegenden Erfindung, je nach ihrem Aufbau, beispielsweise verwendet werden als Hautschutzcrème, Reinigungsmilch, Sonnenschutzlotion, Nährcrème, Tages- oder Nachtcrème usw. Es ist gegebenenfalls möglich und vorteilhaft, die erfindungsgemäßen Zusammensetzungen als Grundlage für pharmazeutische Formulierungen zu verwenden.

Die erfindungsgemäßen kosmetischen und dermatologischen Zubereitungen können kosmetische Hilfsstoffe enthalten, wie sie üblicherweise in solchen Zubereitungen verwendet werden, z. B. Konservierungsmittel, Bakterizide, Parfüme, Substanzen zum Verhindern des Schäumens, Farbstoffe, Pigmente, die eine färbende Wirkung haben, Verdickungsmittel, weichmachende, anfeuchtende und/oder feuchthaltende Substanzen, Fette, Öle, Wachse oder andere übliche Bestandteile einer kosmetischen oder dermatologischen Formulierung wie Alkohole, Polyole, Polymere, Schaumstabilisatoren, Elektrolyte, organische Lösemittel oder Silikonderivate.

Eine erstaunliche Eigenschaft der erfindungsgemäßen Zubereitungen ist, daß diese sehr gute Vehikel für kosmetische oder dermatologische Wirkstoffe in die Haut sind, wobei vorteilhafte Wirkstoffe Antioxidantien sind, welche die Haut vor oxidativer Beanspruchung schützen können.

Erfindungsgemäß enthalten die Zubereitungen vorteilhaft eines oder mehrere Antioxidantien. Als günstige, aber dennoch fakultativ zu verwendende Antioxidantien können alle für kosmetische und/oder dermatologische Anwendungen geeigneten oder gebräuchlichen Antioxidantien verwendet werden. Es ist dabei vorteilhaft, Antioxidantien als einzige Wirkstoffklasse zu verwenden, etwa dann, wenn eine kosmetische oder dermatologische Anwendung im Vordergrund steht, wie z. B. die Bekämpfung der oxidativen Beanspruchung der Haut. Es ist aber auch günstig, die erfindungsgemäßen Stiftzubereitungen mit einem Gehalt an einem oder mehreren Antioxidantien zu versehen, wenn die Zubereitungen einem anderen Zweck dienen sollen, z. B. als Sonnenschutzmittel.

Besonders vorteilhaft werden die Antioxidantien gewählt aus der Gruppe bestehend aus Aminosäuren (z. B. Glycin, Histidin, Tyrosin. Tryptophan) und deren Derivate, Imidazole (z. B. Urocaninsäure) und deren Derivate, Peptide wie D,L-Carnosin, D-Carnosin, L-Carnosin und deren Derivate (z. B. Anserin), Carotinoide, Carotine (z. B. α-Carotin, β-Carotin, Lycopin) und deren Derivate, Chlorogensäure und deren Derivate, Liponsäure und deren Derivate (z. B. Dihydroliponsäure), Aurothioglucose, Propylthiouracil und andere Thiole (z. B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl-, Cholesteryl- und Glycerylester) sowie deren Salze, Dilaurylthiodipropionat, Distearylthiodipropionat Thiodipropionsäure und deren Derivate (Ester, Ether, Peptide, Lipide, Nukleotide, Nukleoside und Salze) sowie Sulfoximinverbindungen (z. B. Buthioninsulfoximine, Homocysteinsulfoximin, Buthioninsulfone, Penta-, Hexa-, Heptathioninsulfoximin) in sehr geringen verträglichen Dosierungen (z. B. pmol bis μmol/kg), ferner (Metall)-Chelatoren (z. B. α-Hydroxyfettsäuren, Palmitinsäure, Phytinsäure, Lactoferrin), α-Hydroxysäuren (z. B. Citronensäure, Milchsäure, Apfelsäure), Huminsäure, Gallensäure, Gallenextrakte, Bilirubin, Biliverdin, EDTA, EGTA und deren Derivate, ungesättigte Fettsäuren und deren Derivate (z. B. γ-Linolensäure, Linolsäure, Ölsäure), Folsäure und deren Derivate, Ubichinon und Ubichinol und deren Derivate, Vitamin C und Derivate (z. B. Ascorbylpalmitat, Mg-Ascorbylphosphat, Ascorbylacetat), Tocopherole und Derivate (z. B. Vitamin-E-acetat). Vitamin A und Derivate (Vitamin-A-palmitat) sowie Koniferylbenzoat des Benzoeharzes, Rutinsäure und deren Derivate, α-Glycosylrutin, Ferulasäure, Furfurylidenglucitol, Carnosin, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Zink und dessen Derivate (z. B. ZnO, $ZnSO_4$), Selen und dessen Derivate (z. B. Selenmethionin), Stilbene und deren Derivate (z. B. Stilbenoxid, Trans-Stilbenoxid) und die erfindungsgemäß geeigneten Derivate (Salze, Ester, Ether. Zucker, Nukleotide, Nukleoside, Peptide und Lipide) dieser genannten Wirkstoffe.

Die Menge der vorgenannten Antioxidantien (eine oder mehrere Verbindungen) in den erfindungsgemäßen Zubereitungen beträgt vorzugsweise 0,001 bis 30 Gew.-%, besonders bevorzugt 0,05 bis 20 Gew.-%, insbesondere 0,1 bis 10 Gew.-%, bezogen auf das Gesamtgewicht der Zubereitung.

Sofern Vitamin E und/oder dessen Derivate das oder die Antioxidantien darstellen, ist vorteilhaft, deren jeweilige Konzentrationen aus dem Bereich von 0,001 bis 10 Gew.-%, bezogen auf das Gesamtgewicht der Formulierung, zu wählen.

Sofern Vitamin A, bzw. Vitamin-A-Derivate, bzw. Carotine bzw. deren Derivate das oder die Antioxidantien darstellen, ist vorteilhaft, deren jeweilige Konzentrationen aus dem Bereich von 0,001 bis 10 Gew.-%, bezogen auf das Gesamtgewicht der Formulierung, zu wählen.

Erfindungsgemäß können die Wirkstoffe (eine oder mehrere Verbindungen) auch sehr vorteilhaft gewählt werden aus der Gruppe der lipophilen Wirkstoffe, insbesondere aus folgender Gruppe:

Acetylsalicylsäure, Atropin, Azulen, Hydrocortison und dessen Derivaten, z. B. Hydrocortison-17-valerat, Vitamine, z. B. Ascorbinsäure und deren Derivate, Vitamine der B- und D-Reihe, sehr günstig das Vitamin $B_1$, das Vitamin $B_{12}$ das Vitamin $D_1$, aber auch Bisabolol, ungesättigte Fettsäuren, namentlich die essentiellen Fettsäuren (oft auch Vitamin F genannt), insbesondere die gamma-Linolensäure, Ölsäure, Eicosapentaënsäute, Docosahexaënsäure und deren Derivate, Chloramphenicol, Coffein, Prostaglandine, Thymol, Campher, Extrakte oder andere Produkte pflanzlicher und tierischer Herkunft, z. B. Nachtkerzenöl, Borretschöl oder Johannisbeerkemöl, Fischöle, Lebertran aber auch Ceramide und ceramidähnliche Verbindungen und so weiter.

Vorteilhaft ist es auch, die Wirkstoffe aus der Gruppe der rückfettenden Substanzen zu wählen, beispielsweise Purcellinöl, Eucerit® und Neocerit®.

Die Liste der genannten Wirkstoffe bzw. Wirkstoffkombinationen, die in den erfindungsgemäßen Pickering-Emulsionen verwendet werden können, soll selbstverständlich nicht limitierend sein.

Günstig sind auch solche kosmetischen und dermatologischen Zubereitungen, die in der Form eines Sonnenschutzmittels vorliegen. Vorzugsweise enthalten diese neben den erfindungsgemäßen Wirkstoffkombinationen zusätzlich mindestens eine UV-A-Filtersubstanz und/oder mindestens eine UV-B-Filtersubstanz. Solche Formulierungen können, obgleich nicht notwendig, gegebenenfalls auch ein oder mehrere anorganische Pigmente als UV-Filtersubstanzen enthalten.

Bevorzugt sind anorganische Pigmente auf Basis von Metalloxiden und/oder anderen in Wasser schwerlöslichen oder unlöslichen Metallverbindungen enthalten, insbesondere der Oxide des Titans ($TiO_2$), Zinks (ZnO), Eisens (z. B. $Fe_2O_3$), Zirkoniums ($ZrO_2$), Siliciums ($SiO_2$), Mangans (z. B. MnO), Aluminiums ($Al_2O_3$), Cers (z. B. $Ce_2O_3$),

Mischoxiden der entsprechenden Metalle sowie Abmischungen aus solchen Oxiden.

Auch ein zusätzlicher Gehalt an stabilisierend wirkenden Titandioxid- und/oder Zinkoxidpartikeln kann selbstverständlich vorteilhaft sein, ist aber im Sinne der vorliegenden Erfindung nicht notwendig.

Es ist auch vorteilhaft im Sinne der vorliegenden Erfindungen, solche kosmetischen und dermatologischen Zubereitungen zu erstellen, deren hauptsächlicher Zweck nicht der Schutz vor Sonnenlicht ist, die aber dennoch einen Gehalt an UV-Schutzsubstanzen enthalten. So werden z. B. in Tagescremes gewöhnlich UV-A- bzw. UV-B-Filtersubstanzen eingearbeitet.

Auch stellen UV-Schutzsubstanzen, ebenso wie Antioxidantien und, gewünschtenfalls, Konservierungsstoffe, einen wirksamen Schutz der Zubereitungen selbst gegen Verderb dar.

Vorteilhaft enthalten erfindungsgemäße Zubereitungen Substanzen, die UV-Strahlung im UV-A- und/oder UV-B-Bereich absorbieren wobei die Gesamtmenge der Filtersubstanzen z. B. 0,1 Gew.-% bis 30 Gew.-%, vorzugsweise 0,5 bis 20 Gew.-%, insbesondere 1,0 bis 15,0 Gew.-% beträgt, bezogen auf das Gesamtgewicht der Zubereitungen, um kosmetische Zubereitungen zur Verfügung zu stellen, die das Haar bzw. die Haut vor dem gesamten Bereich der ultravioletten Strahlung schützen. Sie können auch als Sonnenschutzmittel fürs Haar oder die Haut dienen.

Vorteilhafte UV-A-Filtersubstanzen im Sinne der vorliegenden Erfindung sind Dibenzoylmethanderivate, insbesondere das 4-(tert.-Butyl)-4'-methoxydibenzoylmethan (CAS-Nr. 70356-09-1), welches von Givaudan unter der Marke Parsol® 1789 und von Merck unter der Handelsbezeichnung Eusolex® 9020 verkauft wird.

Weitere vorteilhafte UV-A-Filtersubstanzen sind die Phenylen-1,4-bis-(2-benzimidazyl)-3,3'-5,5'-tetrasulfonsäure:

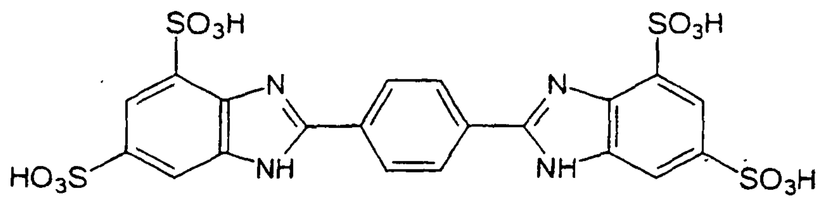

und ihre Salze, besonders die entsprechenden Natrium-, Kalium- oder Triethanolammonium-Salze, insbesondere das Phenylen-1,4-bis-(2-benzimidazyl)-3,3'-5,5'-tetrasulfonsäure-bis-natriumsalz:

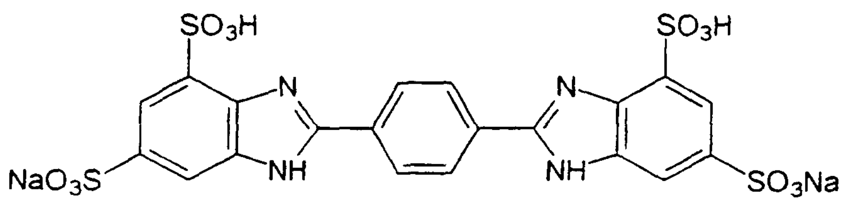

sowie das 1,4-di(2-oxo-10-Sulfo-3-bomylidenmethyl)-Benzol und dessen Salze (besonders die entprechenden 10-Sulfato-verbindungen, insbesondere das entsprechende Natrium-, Kalium- oder Triethanolammonium-Salz), das auch als Benzol-1,4-di(2-oxo-3-bornylidenmethyl-10-sulfonsäure) bezeichnet wird und sich durch die folgende Struktur auszeichnet:

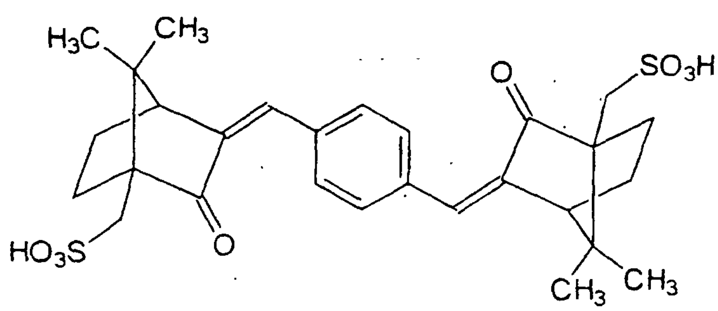

Vorteilhafte UV-Filtersubstanzen im Sinne der vorliegenden Erfindung sind ferner sogenannte Breitbandfilter, d.h. Filtersubstanzen, die sowohl UV-A- als auch UV-B-Strahlung absorbieren.

Vorteilhafte Breitbandfilter oder UV-B-Filtersubstanzen sind beispielsweise Bis-Resorcinyltriazinderivate mit der folgenden Struktur

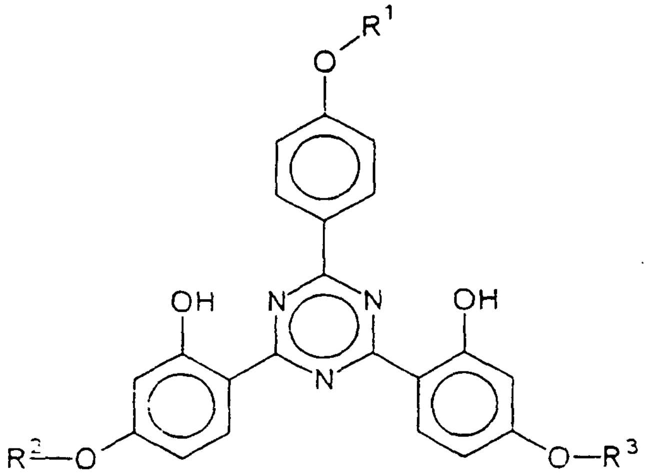

wobei $R^1$, $R^2$ und $R^3$ unabhangig voneinander gewählt werden aus der Gruppe der verzweigten und unverzweigten Alkylgruppen mit 1 bis 10 Kohlenstoffatomen bzw. ein einzelnes Wasserstoffatom darstellen Insbesondere bevorzugt sind das 2,4-Bis-{[4-(2-Ethylhexyloxy)-2-hydroxy]-phenyl}-6-(4-methoxyphenyl)-1,3,5-triazin (INCI: Aniso Triazin), welches unter der Handelsbezeichnung Tinosorb® S bei der CIBA-Chemikalien GmbH erhältlich ist, und das 4,4',4"-(1,3,5-Triazin-2,4,6-triyltriimino)-tris-benzoësäure-tris(2-ethylhexylester), synonym: 2,4,6-Tris-[anilino-(p-carbo-2'-ethyl-1'-hexyloxy)]-1,3,5-triazin (INCI: Octyl Triazone), welches von der BASF Aktiengesellschaft unter der Warenbezeichnung UVINUL® T 150 vertrieben wird

Auch andere UV-Filtersubstanzen beschrieben welche das Strukturmotiv

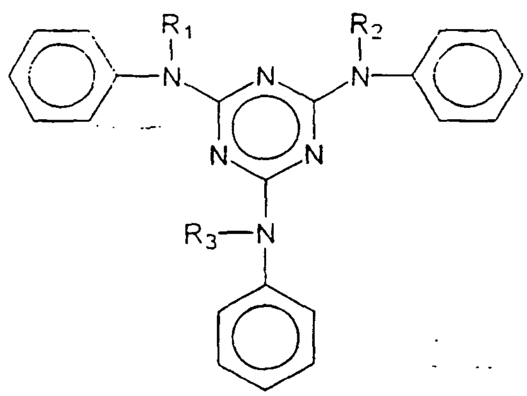

aufweisen, sind vorteilhafte UV-Filtersubstanzen im Sinne der vorliegenden Erfindung, beispielsweise die in der Europäischen Offenlegungsschrift EP 570 838 A1 beschriebenen s-Triazindenvate, deren chemische Struktur durch die generische Formel

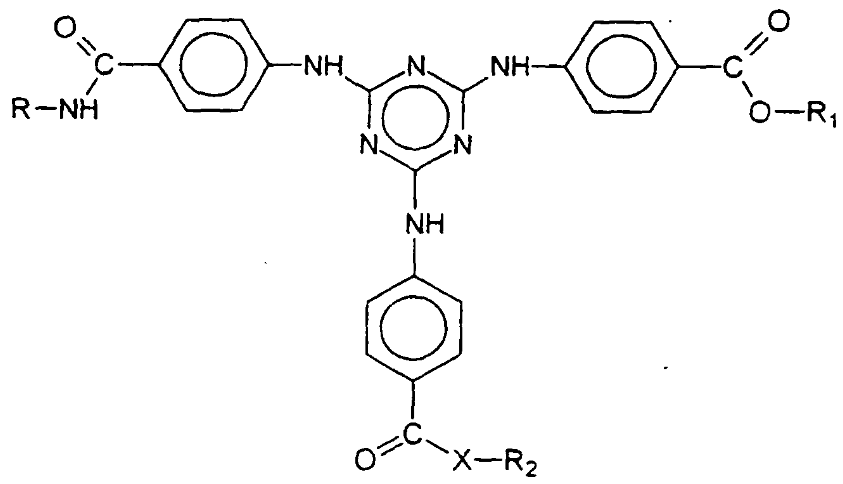

wiedergegeben wird, wobei

R einen verzweigten oder unverzweigten $C_1$-$C_{18}$-Alkylrest, einen $C_5$-$C_{12}$-Cycloalkylrest, gegebenenfalls substituiert mit einer oder mehreren $C_1$-$C_4$- Alkylgruppen, darstellt,

X ein Sauerstoffatom oder eine NH-Gruppe darstellt,

$R_1$ einen verzweigten oder unverzweigten $C_1$-$C_{18}$-Alkylrest, einen $C_5$-$C_{12}$-Cycloalkylrest, gegebenenfalls substituiert mit einer oder mehreren $C_1$-$C_4$- Alkylgruppen, oder ein Wasserstoffatom, ein Alkalimetallatom, eine Ammoniumgruppe oder eine Gruppe der Formel

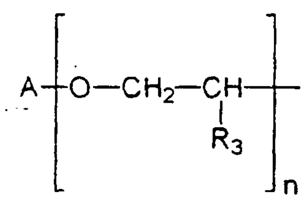

bedeutet, in welcher

A einen verzweigten oder unverzweigten $C_1$-$C_{18}$-Alkylrest, einen $C_5$-$C_{12}$-Cycloalkyl - oder Arylrest darstellt, gegebenenfalls substituiert mit einer oder mehreren $C_1$-$C_4$- Alkylgruppen,

$R_3$ ein Wasserstoffatom oder eine Methylgruppe darstellt,

n eine Zahl von 1 bis 10 darstellt,

$R_2$ einen verzweigten oder unverzweigten $C_1$-$C_{18}$-Alkylrest, einen $C_5$-$C_{12}$-Cycloalkylrest, gegebenenfalls substituiert mit einer oder mehreren $C_1$-$C_4$- Alkylgruppen, darstellt, wenn X die NH-Gruppe darstellt, und einen verzweigten oder unverzweigten $C_1$-$C_{18}$-Alkylrest, einen $C_5$-$C_{12}$-Cycloalkylrest, gegebenenfalls substituiert mit einer oder mehreren $C_1$-$C_4$- Alkylgruppen, oder ein Wasserstoffatom, ein Alkalimetallatom, eine Ammoniumgruppe oder eine Gruppe der Formel

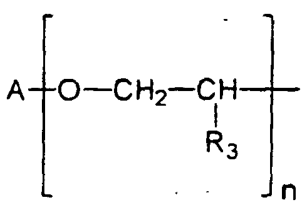

bedeutet, in welcher

A einen verzweigten oder unverzweigten $C_1$-$C_{18}$-Alkylrest, einen $C_5$-$C_{12}$-Cycloalkyl - oder Arylrest darstellt, gegebenenfalls substituiert mit einer oder mehreren $C_1$-$C_4$- Alkylgruppen,

$R_3$ ein Wasserstoffatom oder eine Methylgruppe darstellt,

n eine Zahl von 1 bis 10 darstellt,

wenn X ein Sauerstoffatom darstellt.

Besonders bevorzugte UV-Filtersubstanz im Sinne der vorliegenden Erfindung ist ferner ein unsymmetrisch substituiertes s-Triazin, dessen chemische Struktur durch die Formel

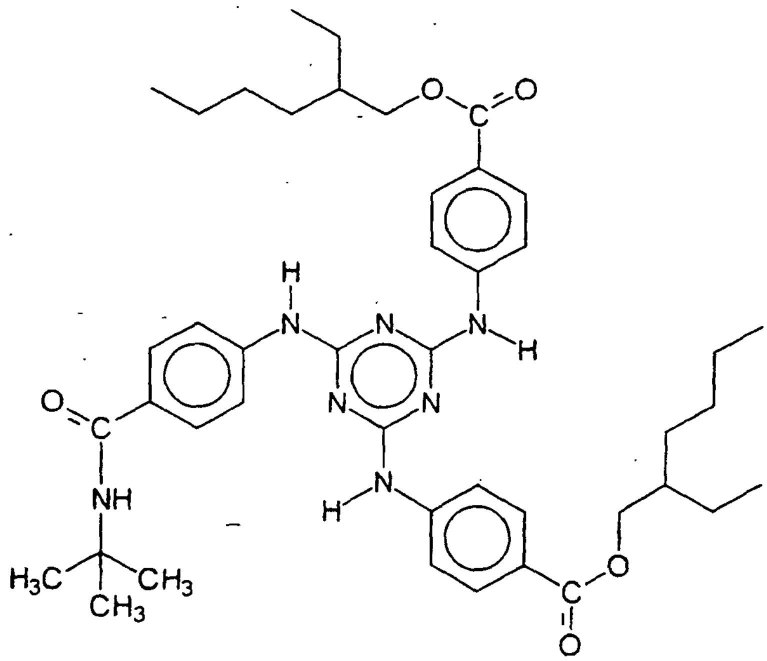

wiedergegeben wird, welche im Folgenden auch als Dioctylbutylamidotriazon (INCI: Dioctylbutamidotriazone) bezeichnet wird und unter der Handelsbezeichnung UVASORB HEB bei Sigma 3V erhältlich ist.

Auch in der Europäischen Offenlegungsschrift 775 698 werden bevorzugt einzusetzende Bis-Resorcinyltriazinderivate beschrieben, deren chemische Struktur durch die generische Formel

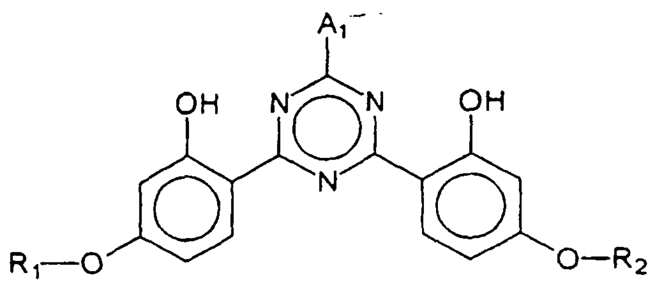

wiedergegeben wird, wobei $R_1$ , $R_2$ und $A_1$ verschiedenste organische Reste repräsentieren.

Vorteilhaft im Sinne der vorliegenden Erfindung sind ferner das 2,4-Bis-{[4-(3-sulfonato)-2-hydroxy-propyloxy)-2-hydroxy]-phenyl}-6-(4-methoxyphenyl)-1,3,5-triazin Natriumsalz, das 2,4-Bis-{[4-(3-(2-Propyloxy)-2-hydroxy-propyloxy)-2-hydroxy]-phenyl}-6-(4-methoxyphenyl)-1,3,5-triazin, das 2,4-Bis-{[4-(2-ethyl-hexyloxy)-2-hydroxy]-phenyl}-6-[4-(2-methoxyethylcarboxyl)-phenylamino]-1,3,5-triazin, das 2,4-Bis-{[4-(3-(2-propyloxy)-2-hydroxy-propyloxy)-2-hydroxy]-phenyl}-6-[4-(2-ethyl-carboxyl)-phenylamino]-1,3,5-triazin, das 2,4-Bis-{[4-(2-ethyl-hexyloxy)-2-hydroxy]-phenyl}-6-(1-methyl-pyrrol-2-yl)-1,3,5-triazin, das 2,4-Bis-{[4-tris(trimethylsiloxy-silylpropyloxy)-2-hydroxy]-phenyl}-6-(4-methoxyphenyl)-1,3,5-triazin, das 2,4-Bis-{[4-(2"-methylpropenyloxy)-2-hydroxy]-phenyl}-6-(4-methoxyphenyl)-1,3,5-triazin und das 2,4-Bis-{[4-(1',1',1',3',5',5',5'-Heptamethylsiloxy-2"-methyl-propyloxy)-2-hydroxy]-phenyl}-6-(4-methoxyphenyl)-1,3,5-triazin.

Ein vorteilhafter Breitbandfilter im Sinne der vorliegenden Erfindung ist das 2,2'-Methylen-bis-(6-(2H-benzotriazol-2-yl)-4-(1,1,3,3-tetramethylbutyl)-phenol), welches durch die chemische Strukturformel

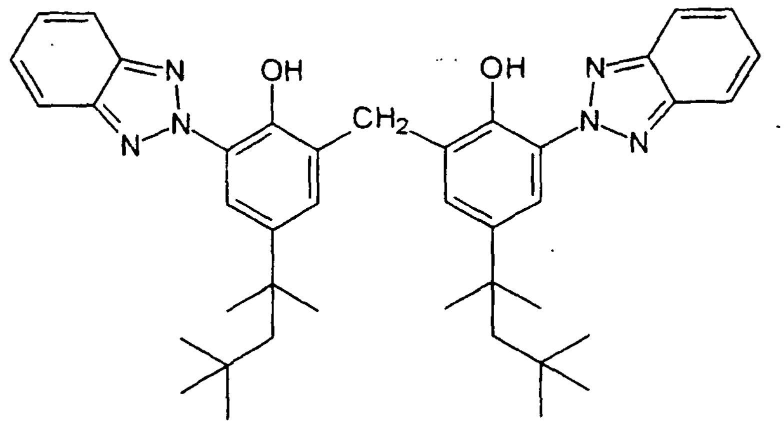

gekennzeichnet ist und unter der Handelsbezeichnung Tinosorb® M bei der CIBA-Chemikalien GmbH erhältlich ist.

Vorteilhafter Breitbandfilter im Sinne der vorliegenden Erfindung ist ferner das 2-(2H-benzotriazol-2-yl)-4-methyl-6-[2-methyl-3-[1,3,3,3-tetramethyl-1-[(trimethylsilyl)oxy]disiloxanyl]propyl]-phenol (CAS-Nr.: 155633-54-8) mit der INCI-Bezeichnung Drometrizole Trisiloxane, welches durch die chemische Strukturformel

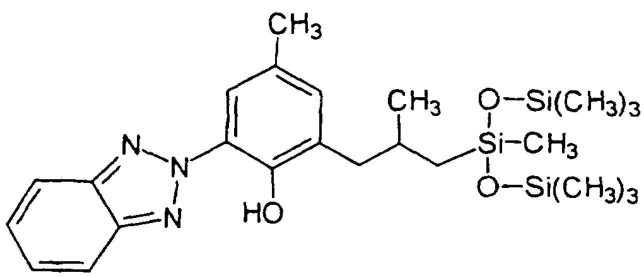

gekennzeichnet ist.

Die UV-B-Filter können öllöslich oder wasserlöslich sein. Vorteilhafte öllösliche UV-B-Filtersubstanzen sind z. B.:

- 3-Benzylidencampher-Derivate, vorzugsweise 3-(4-Methylbenzyliden)campher, 3-Benzylidencampher;
- 4-Aminobenzoesäure-Derivate, vorzugsweise 4-(Dimethylamino)-benzoesäure(2-ethylhexyl)ester, 4-(Dimethylamino)benzoesäureamylester;
- 2,4,6-Trianilino-(p-carbo-2'-ethyl-1'-hexyloxy)-1,3,5-triazin;
- Ester der Benzalmalonsäure, vorzugsweise 4-Methoxybenzalmalonsäuredi(2-ethylhexyl)ester;
- Ester der Zimtsäure, vorzugsweise 4-Methoxyzimtsäure(2-ethylhexyl)ester, 4-Methoxyzimtsäureisopentylester;
- Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon
- sowie an Polymere gebundene UV-Filter.

Vorteilhafte wasserlösliche UV-B-Filtersubstanzen sind z. B.:

- Salze der 2-Phenylbenzimidazol-5-sulfonsäure, wie ihr Natrium-, Kalium- oder ihr Triethanolammonium-Salz, sowie die Sulfonsäure selbst;
- Sulfonsäure-Derivate des 3-Benzylidencamphers, wie z. B. 4-(2-Oxo-3-bornylidenmethyl)benzolsulfonsäure, 2-Methyl-5-(2-oxo-3-bornylidenmethyl)sulfonsäure und deren Salze.

Eine weiterere erfindungsgemäß vorteilhaft zu verwendende Lichtschutzfiltersubstanz ist das Ethylhexyl-2-cyano-3.3-diphenylacrylat (Octocrylen), welches von BASF unter der Bezeichnung Uvinul® N 539 erhältlich ist und

sich durch folgende Struktur auszeichnet:

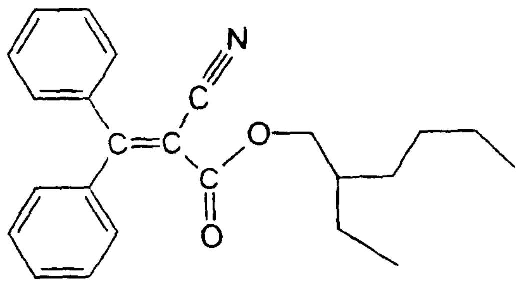

Es kann auch von erheblichem Vorteil sein, polymergebundene oder polymere UV-Filtersubstanzen in Zubereitungen gemäß der vorliegenden Erfindung zu verwenden, insbesondere solche, wie sie in der WO-A-92/20690 beschrieben werden.

Ferner kann es gegebenenfalls von Vorteil sein, erfindungsgemäß weitere UV-A- und/oder UV-B-Filter in kosmetische oder dermatologische Zubereitungen einzuarbeiten, beispielsweise bestimmte Salicylsäurederivate wie 4-Isopropylbenzylsalicylat, 2-Ethylhexylsalicylat (= Octylsalicylat), Homomenthylsalicylat.

Die Liste der genannten UV-Filter die im Sinne der vorliegenden Erfindung eingesetzt werden können, soll selbstverständlich nicht limitierend sein.

Die nachfolgenden Beispiele sollen die vorliegende Erfindung verdeutlichen, ohne sie einzuschränken. Die Zahlenwerte in den Beispielen bedeuten Gewichtsprozente, bezogen auf das Gesamtgewicht der jeweiligen Zubereitungen.

**Beispiele:**

| | **1** | **2** | **3** | **4** | **5** | **6** | **7** |
|---|---|---|---|---|---|---|---|
| Bentone 38 ® | 0,5 | 1 | 0,2 | | 0,8 | | 0,5 |
| Bentone 27 ® | | | 0,3 | 0,7 | | 0,3 | |
| Polyglyceryl-2 Dipolyhydroxystearat | | | | | 0,2 | | |
| Cetyl Dimethicon Copolyol | | | | | | 0,3 | |
| PEG-30 Dipolyhydroxystearat | | | | | | | 0,2 |
| Caprylic/Capric Triglycerid | 10 | 5 | | 8 | | 5 | |
| Octyldodecanol | | | 10 | 10 | | 5 | |
| Dicaprylylether | 5 | 5 | 5 | | | 5 | |
| Cetearylisononanoat | | | 2 | | | | |
| Dimethicone | 2 | 1 | | 2 | 5 | | 5 |
| Mineralöl Isohexadekan | | 5 | | | 5 | 5 | 2 |
| | | 2 | | | 5 | | |
| Hydriertes Polyisobuten | 2 | | | 5 | | 5 | |
| Butylen Glycol Dicaprylat/Caprat | | 2 | 5 | 5 | 5 | | 8 |
| $C_{12-15}$ Alkylbenzoat | 7 | 3 | | 5 | | | 5 |
| Vitamen E-Acetat | 0,5 | 0,5 | 0,5 | | | 0,5 | |
| Dioctyl Butamido Triazon | | | | | 3 | 1 | |
| Octocrylen | | 5 | 8 | | | 6 | 5 |
| Octyltriazon | 1 | | 4 | | | | |

(fortgesetzt)

| | 1 | 2 | 3 | 4 | 5 | 6 | 7 |
|---|---|---|---|---|---|---|---|
| Methylbenzyliden Campher | 4 | 4 | 4 | | 2 | | 4 |
| Butylmethoxydibenzoylmethan | 2 | | 3 | | 2 | | 2 |
| Titandioxid | | | | | 5 4 | | 2 |
| Aerosil R972 ® | | 0,5 | | | | 0,5 | |
| Konservierung | 0,5 | 0,5 | 0,5 | 0,5 | 0,5 | 0,5 | 0,5 |
| α-Glucosylrutin | 0,5 | 0,1 | 0,2 | 0,2 | 0,5 | 0,1 | 0,7 |
| Glycerin | 3 | 5 | 10 | 10 | 5 | 10 | 5 |
| $MgSO_4$ | | | | | 1 | 1 | |
| NaCl | 0,5 | 1 | 0,2 | | | | 1 |
| Phenylbenzimidazolsulfonsäure | | | 2 | | | | 4 |
| Natronlauge 45% | | | 0,8 | | | | 1,3 |
| Wasser | ad100 | ad100 | ad100 | ad100 | ad100 | ad100 | ad100 |

**Patentansprüche**

**1.** Kosmetische oder dermatologische Zubereitungen, die feindisperse Systeme vom Typ Wasser-in-Öl darstellen, enthaltend

1. eine Ölphase,
2. eine Wasserphase,
3. mindestens ein modifiziertes Schichtsilikat, welches sowohl hydrophile als auch lipophile Eigenschaften zeigt, welches also amphiphilen Charakter besitzt und sich an der Grenzflache Wasser/Öl anordnet,
4. mindestens ein Flavon. Flavanon und/oder Flavonoid und
5. höchstens 0,5 Gew. % eines oder mehrerer Emulgatoren

**2.** Zubereitungen nach Anspruch 1, **dadurch gekennzeichnet, daß** sie emulgatorfrei sind.

**3.** Zubereitungen nach einem der vorhergehenden Ansprüche, **dadurch gekennzeichnet, daß** weitere kosmetische oder pharmazeutische Hilfs-, Zusatz- und/oder Wirkstoffe enthalten sind.

**4.** Zubereitungen nach einem der vorhergehenden Ansprüche, **dadurch gekennzeichnet, daß** der Gehalt an einem oder mehreren modifizierten Schichtsilikaten zwischen 0,05 Gew.-% und 20 Gew.-% ist, vorteilhaft zwischen 0.1 und 5 Gew.-%, bezogen auf das Gesamtgewicht der Zubereitungen.

**5.** Zubereitungen nach einem der vorhergehenden Ansprüche, **dadurch gekennzeichnet, daß** der Gehalt an einem oder mehreren Flavonen, Flavanonen und/oder Flavonoiden aus dem Bereich von 0,01 bis 5,0 Gew.-%, bevorzugt 0,1 bis 2,0 Gew.-% gewählt wird, bezogen auf das Gesamtgewicht der Zubereitungen

**6.** Zubereitungen nach einem der vorhergehenden Ansprüche, **dadurch gekennzeichnet, daß** neben einem oder mehreren modifizierten Schichtsilikaten weitere Pigmente enthalten sind, insbesondere Bomitrid und/oder modifizierte Polysaccharide und/oder mikrofeine Polymerpartikel und/oder mikronisierte, anorganische Pigmente, die gewählt werden aus der Gruppe der amphiphilen Metalloxide, insbesondere aus der Gruppe Titandioxid, Zinkoxid, Eisenoxide bzw. Eisenmischoxide, Siliciumdioxid bzw. Silicate, wobei die Pigmente sowohl einzeln als auch im Gemisch vorliegen können.

**7.** Zubereitungen nach einem der vorhergehenden Ansprüche, enthaltend einen oder mehrere Zusatz- oder Wirkstoffe, gewählt aus der Gruppe der Antioxidantien und/oder der Gruppe der UV-Filtersubstanzen.

## Claims

1. Cosmetic or dermatological preparations, which are finely disperse systems of the water-in-oil type, comprising

   1. an oil phase,
   2. a water phase,
   3. at least one modified phyllosilicate, which exhibits both hydrophilic and lipophilic properties, i.e. which has amphiphilic character and positions itself at the water/oil interface,
   4. at least one flavone, flavanone and/or flavonoid and
   5. at most 0.5% by weight of one or more emulsifiers.

2. Preparations according to Claim 1, **characterized in that** they are emulsifier-free.

3. Preparations according to one of the preceding claims, **characterized in that** further cosmetic or pharmaceutical auxiliaries, additives and/or active ingredients are present.

4. Preparations according to one of the preceding claims, **characterized in that** the content of one or more modified phyllosilicates is between 0.05% by weight and 20% by weight, advantageously between 0.1 and 5% by weight, based on the total weight of the preparations.

5. Preparations according to one of the preceding claims, **characterized in that** the content of one or more flavones, flavanones and/or flavonoids is chosen from the range from 0.01 to 5.0% by weight, preferably 0.1 to 2.0% by weight, based on the total weight of the preparations.

6. Preparations according to one of the preceding claims, **characterized in that**, in addition to one or more modified phyllosilicates, further pigments are present, in particular boron nitride and/or modified polysaccharides and/or microfine polymer particles and/or micronized, inorganic pigments which are chosen from the group of amphiphilic metal oxides, in particular from the group consisting of titanium dioxide, zinc oxide, iron oxides or iron mixed oxides, silicon dioxide or silicates, it being possible for the pigments to be present individually or as a mixture.

7. Preparations according to one of the preceding claims, comprising one or more additives or active ingredients chosen from the group of antioxidants and/or the group of UV filter substances.

## Revendications

1. Préparations cosmétiques ou dermatologiques, qui représentent des systèmes finement dispersés du type eau-dans-huile, contenant

   1. une phase huileuse,
   2. une phase aqueuse,
   3. au moins un silicate lamellaire modifié qui présente aussi bien des propriétés hydrophiles que des propriétés lipophiles, qui a donc un caractère amphiphile et se dispose à l'interface eau/huile,
   4. au moins une flavone, une flavanone et/ou un flavonoïde et
   5. au maximum 0,5 % en poids d'un ou plusieurs émulsifiants.

2. Préparations selon la revendication 1, **caractérisées en ce qu'**elles sont exemptes d'émulsifiant.

3. Préparations selon l'une quelconque des revendications précédentes, **caractérisées en ce que** d'autres adjuvants, additifs et/ou actifs cosmétiques ou pharmaceutiques sont contenus.

4. Préparations selon l'une quelconque des revendications précédentes, **caractérisées en ce que** la teneur en un ou plusieurs silicates lamellaires modifiés est comprise entre 0,05 % en poids et 20 % en poids, avantageusement entre 0,1 et 5,0 % en poids, par rapport au poids total des préparations.

5. Préparations selon l'une quelconque des revendications précédentes, **caractérisées en ce que** la teneur en une ou plusieurs flavones, flavanones et/ou un ou plusieurs flavonoïdes est choisie dans la plage de 0,01 à 5,0 % en poids, de préférence de 0,1 à 2,0 % en poids, par rapport au poids total des préparations.

6. Préparations selon l'une quelconque des revendications précédentes, **caractérisées en ce que**, outre un ou plusieurs silicates lamellaires modifiés, d'autres pigments sont contenus, en particulier du nitrure de bore et/ou des polysaccharides modifiés et/ou des particules microfines de polymère et/ou des pigments minéraux micronisés qui sont choisis dans le groupe des oxydes métalliques amphiphiles, en particulier dans le groupe constitué par le dioxyde de titane, l'oxyde de zinc, les oxydes de fer ou oxydes mixtes de fer, le dioxyde de silicium ou les silicates, les pigments pouvant être présents aussi bien individuellement qu'en mélange.

7. Préparations selon l'une quelconque des revendications précédentes, contenant un ou plusieurs actifs ou additifs, choisis dans le groupe des antioxydants et/ou dans le groupe des filtres UV.